# EUROPÄISCHE PATENTSCHRIFT

(11) **EP 1 945 784 B1**
(45) Veröffentlichungstag und Bekanntmachung des Hinweises auf die Patenterteilung: **08.08.2012**
(21) Anmeldenummer: 06806492.2
(22) Anmeldetag: 24.10.2006
(51) Int. Cl.: C12P 3/00, A61K 8/22

(54) **SYSTEM ZUR ENZYMATISCHEN GENERIERUNG VON WASSERSTOFFPEROXID**
SYSTEM FOR ENZYMATICALLY GENERATING HYDROGEN PEROXIDE
SYSTEME POUR PRODUIRE DU PEROXYDE D'HYDROGENE PAR VOIE ENZYMATIQUE

(30) Priorität: 08.11.2005 DE 102005053529
(43) Veröffentlichungstag der Anmeldung: 23.07.2008
(73) Patentinhaber: Henkel AG & Co. KGaA, 40589 Düsseldorf (DE)
(72) Erfinder: WEBER, Thomas, 40589 Düsseldorf (DE); HOVEN, Nina, 40223 Düsseldorf (DE); MAURER, Karl-Heinz, 40699 Erkrath (DE)
(86) Internationale Anmeldenummer: PCT/EP2006/010226
(87) Internationale Veröffentlichungsnummer: WO 2007/054203

(56) Entgegenhaltungen:
- EP-A- 1 559 412
- EP-A1- 0 242 007
- EP-A1- 0 310 675
- WO-A-02/32384
- WO-A-02/099023
- WO-A-2004/024859
- WO-A-2004/039345
- WO-A-2004/060324
- WO-A-2005/124012
- DATABASE EMBL [Online] 10. Mai 2005 (2005-05-10), XP002431083 Database accession no. Q9ZBU1 -& DATABASE EMBL [Online] 16. April 2005 (2005-04-16), XP002431234 Database accession no. AL939126
- DATABASE EMBL [Online] zur Patentschrift JP 10 075785 A gehörend 9. September 1998 (1998-09-09), XP002431481 Database accession no. AAV11297 -& DATABASE WPI Week 199822 Derwent Publications Ltd., London, GB; AN 1998-244359 XP002431042 & JP 10 075785 A (IKEDA SHOKKEN KK) 24. März 1998 (1998-03-24)
- HIGARA, K. ET AL.: "Molecular Cloning and Expression of a Gene Encoding a Novel Sorbitol Oxidase from Streptomyces sp. H-7775" BIOSCIENCE, BIOTECHNOLOGY, AND BIOCHEMISTRY, Bd. 62, Nr. 2, Februar 1998 (1998-02), Seiten 347-353, XP002431345

## Beschreibung

Die vorliegende Erfindung betrifft Körperpflegemittel, Haarpflegemittel. Haarwaschmiltel, Sußigkeiten, Mund-, Zahn- und Zahnprothesenpflegemittel, Zahnspangenpflegemittel. Kosmetika, Therapeutika, Waschmittel, Reinigungsmittel, Aufhellungsmittel, Bleichmittel, Desinfektionsmittel, Nachspülmittel, Handwaschmittel, Handgeschirrspülmittel, Maschinengeschirrspülmittel und Mittel zur bleichenden oder desinfizierenden Behandlung von Filtermedien, Textilien. Pelzen, Papier, Fellen oder Leder, enthaltend eine Polyol-Oxidase, insbesondere eine Zuckeralkoholoxidase.

Wasserstoffperoxid wird zur Bleiche, Konservierung, Desinfektion (z.B. in Waschmitteln bei der Stoff-, Papier- und Zellstoffbearbeitung, zur Zahnbleiche, für Oberflächen wie Fugen zwischen Kacheln, u. a.) eingeselzt.

Für die herkömmliche chemische Bleiche werden anorganische, alkalische Wasserstoffperoxidlieferanten wie Percarbonat oder Perborat in Kombination mit Bleichboostem (z.B. TAED) eingesetzt. Die Bleichkomponente Wasserstoffperoxid entsteht durch spontanen Zerfall der Additionsverbindung und führt damit schnell aber kurzfristig zu hohen Konzentrationen. Eine schonende Bleiche ist nicht möglich.

Für die Haarfärbung wird herkömmlich eine Bleiche zur Nivellierung von Grautönen vor der Färbung mit Wasserstoffperoxid und Ammoniaklosung durchgeführt. Die kurzfristig hohen Wasserstoffperoxidkonzentrationen in Kombination mit dem alkalischen pH fuhren zu einer deutlichen Haarschädigung.

Es ist bekannt, daß Oxidasen (Alkoholoxidasen, Aminosäureoxidasen) zusammen mit ihren Substraten für die Wasserstoffperoxidgenerierung zur Bleiche und Farbübertragungsinhiblerung in Waschmitteln oder zur enzymatischen Haadärbung und Bleiche in kosmetischen Mitteln eingesetzt werden können.

In der WO 97/21796 wird beschrieben, daß Oxidasen aus ihren entsprechenden Substraten unter technischen Bedingungen (z.B. einer Waschmittelmatrix) mit Hilfe von Luftsauerstoff Wasserstoffperoxid freisetzen und damit zur Bleiche eingeseizt werden können. Die Bildung von Wasserstoffperoxid erfolgt kontinuierlich- wobei die Effizienz der Produktbildung durch die Temperatur- und pH-Stabilität, sowie die. Toleranz gegenüber Substrat und Produkt bestimmt wird.

Die enzymatische Erzeugung von Wasserstoffperoxid hat gegenüber der direkten Beigabe zum Produkt u.a. den Vorteil. daß die Substanz erst unmittelbar bei der Anwendung erzeugt wird, Lagerinstabilität vermieden wird, Verlust des Wasserstoffperoxids während der Lagerung durch Abbau vermieden wird, und daß kein Wasserstoffperoxid als gezepturbestandteil deklariert werden muß. Weiterer Vorteil ist die kontinulediche Nachproduktion von Wasserstoffperoxid, während dieses durch seine Aktivität dem Gleichgewicht entzogen wird.

In der deutschen Patentanmeldung 102 60 930.6-41 der Anmelderin wird dargelegt, daß und wie sich aus den Bakterien Arthrobacter nicotianiae und Arthrobacter aurescens Cholinoxidasen isolieren lassen, die in Bleichsystemen eingesetzt werden können.

Cholinoxidase setzt allerdings bevorzugt Cholin um. Ihre Spezifität nimmt von Cholin über Bis(2-hydroxyethyl) dimethylammoniumchlorid (Dimethyldiethanolammoniumchlorid, DMDEA) und Triethanolamin (TEA) nach Tris (2-hydroxyethyl) methylammoniumchlorid (MTEA) hin ab.

Weitere Polyol-Oxidase/Polyol-Systeme sind bereits im Stand der Technik beschrieben worden. So werden in WO 04/039345 und WO 04/060324 ein Xylitol-Oxidase/Xylitol-System, in WO 04/024859 ein Mannitol-Oxidase/Manitol-System, in WO 04/024859, EP 1559412 und WO 02/099023 ein Polyvinylalkoholoxidaxe/Polivinylalkohol-System sowie in WO 02/32364. EP 0242007 und EP 0310675 ein Glycerinoxidase/Glycerin-System beschrieben. Insbesondere wurden hierbei auch der Einsatz dieser Systeme in Präparaten für medizinische, zahnärztliche oder kosmetische Zwecke und/oder in Reinigungsmittelgemischen beschrieben.

Generell ist die Verfügbarkeit von Enzymen mit unterschiedlicher Substratspezifität wünschenswert. Je nach Fragestellung kann so in einem Prozess auf das bevorzugte Substrat zurückgegriffen werden.

Der vorliegenden Erfindung liegt daher die Augabe zugrunde, ein weiteres enzymbasiertes System zur Generierung von Wasserstoffperoxid beziehungsweise Mittel, die ein solches System enthalten, bereitzustellen.

Diese Aufgabe wurde erfindungsgemäß gelöst durch die Bereitstellung von Korperpflegemitteln, Haarpflegemitteln, Haarwaschmitteln, Süßigkeiten, Mund-, Zahn- und Zahnprothesenpflegemitteln, Zahnspangenpflegemitteln, Kosmelika, Therapeutika, Waschmitteln, Reinigungsmitteln, Aufhellungsmitteln; Bleichmitteln, Desinfektionsmitteln, Nachspülmitteln, Handwaschmitteln, Handgeschirrspülmitteln, Maschinengeschirrspülmitteln und Mitteln zur bleichenden oder desinfizierenden Behandlung von Fillermedien, Textilien, Pelzen, Papier, Fellen oder Leder, enthaltend eine Polyol-Oxidase, insbesondere eine Zuckeralkoholoxidase, deren Aminosauresequenz mit der in Seq. 4 angegebenen Aminosäuresequenz zu mindestens 85 %, insbesondere mindestens 90 %, besonders bevorzugt mindestens 95 % und ganz besonders bevorzugt zu 100 % übereinstimmt.

Zur enzymatischen Generierung von Wasserstoffperoxid enthalten erfindungsgemäße Mittel neben der Polyol-Oxidase vorzugsweise mindestens ein Polyol als Substrat.

Die erfindungsgemäß einsetzbare Polyol-Oxidase ist vorzugsweise ausgewählt unter Zuckeralkohol-Oxidasen, insbesondere unter Mannitoloxidasen, Sorbitoloxidasen, Xylitoloxidasen und Arabitoloxidasen, Desweiteren sind Polyvinylalkoholoxidasen und Glyceroloxidasen unter den bevorzugt ausgewählten Oxidasen.

Eine erfindungsgemaße Zuckeralkoholoxidase Ist beispielsweise erhältlich durch ein- oder mehrfachen Aminosäureaustausch, insbesondere konservativen Aminosäurenaustausch, aus einer Zuckeralkoholoxidase gemäß Seq. 4, oder durch Derivatisierung, Fragmentierung, Deletionsmutation oder Insertionsmutation einer Zuckeralkoholoxidase gemäß Seq. 4.

Bevorzugte Substrate sind ausgewählt unter
a) Zuckeralkoholen, insbesondere unter Mannitol, Sorbitol, Xylitol, Mattilol, Lactitol, isomalt, Galactitol, Arabitol, Ribitol, Erythritol, Adonitol und Iditol, Glycerol und Ethylenglycol, besonders unter D-Sorbitol, D-Xylitol oder D-Mannitol, ganz besonders unter D-Sorbital,
b) Cycliten, insbesondere unter myo-inosil,
c) mehrfachen Akoholen, insbesondere unter Ethylenglycol, 1,4-Butandiol, Polyvinylalkohol oder Glycerol,
d) Pseudozuckern, insbesondere unter Pseudopyranosen und Pseudofuranosen, vorzugsweise unter Valiolamin und Valienamin,
e) Substraten der chemischen Zusammensetzung R¹-[-(CHOH)ₙ-(CH2)ₘ-]ₚ-R², wobei R¹ und R² unabhängig voneinander ausgewählt sind unter H, linearen oder verzweigten, substituierten oder unsubstituierten Alkyl, Alkenyl-, Heteroalkyl-, Alkinyl-, Aryl-, Alkylaryl-, Alkylheteroaryl- und Heleroaryl-Gruppen; wobei p jede ganze Zahl im Bereich von 1 bis 10000, bevorzugt im Bereich von 100 bis 5000 sein kann, m jede ganze Zahl im Bereich von 0 bis 100, bevorzugt im Bereich von 0 bis 10 sein kann und n jede ganze Zahl im Bereich von 1 bis 10 sein kann,
f) modifizierten Substraten nach e), wobei die Alkoholfunktionen zu 0 bis 100%, bevorzugt zu 0 bis 40% und besonders bevorzugt zu 0 bis 10% mit Gruppen der Formel R³-CO- Ester formen oder mit Gruppen der Formel R³- Ether bilden, wobei R³ ausgewählt sein kann unter linearen oder verzweigten, substituierten oder unsubstitulerten Alkyl-, Alkenyl-, Heteroalkyl-, Alkinyl-; Aryl-, Alkylaryl-, Alkylhateroaryl- und Heteroaryl-Gruppen und wobei Ester kurzkettiger Carbonsäuren bevorzugt und Acetat-Gruppen besonders bevorzugt sind, oder
g) Substraten nach e) oder f), Weiterhin dadurch modifiziert, daß solche Polyole oder Polyol-Derivate miteinander kreuzvemetzt sind, indem die Alkohollunktionen der jeweils zu vernetzenden Moleküle beispielsweise durch C2-Dicarbonsäuren oder längerkeitige Dicarbonsäuren Ober zwei Esterbindungen verbunden sind, oder aber durch andere Kreuzvemetzungen von Alkohollunktionen.

Der Begriff Pseudozucker steht für eine Klasse von Verbindungen, in welchen der Ringsauerstoff eines Zuckers durch eine Melhylengruppe ersetzt ist. Pseudozucker kommen in freier Form in der Kulturbrühe von Mikroorganismen, vor allem aber als Komponenten von Pseudooligosacchariden als Enzym-Inhibitoren und Antibiotika vor. Zwei Typen werden unterschieden. Pseudopyranosen und Pseudofurariosen. Einige biologisch aktive Pseudozucker werden in der Natur gefunden,

Beispiele sind Valiolamin und Valienamin, Bestandteile des Trisaccharid-Antibiotikums Validamycin A bzw. der Acarbose.

Das in erfindungsgemäßen Mitteln eingesetzte System zur Generierung von Wasserstoffperoxid weist eine Reihe von Vorteilen gegenüber den aus dem Stand der Technik bekannten Systemen auf:
Beispielsweise ist es besonders zum Einsatz in der Mundhöhle, z. B. bei der Zahnbleiche geeignet, da die Substrate von Zyckeralkohol-Oxidasen in der Regel nicht karfogen oder sogar Kariesvorbeugend sind (im Falle von Xylitol, welches die Nutzung von Glucose durch Plaquebakterien vermindert). Die Produkte der Reaktion von Zuckeralkoholoxidasen sind Aldehyde oder Ketone, aber keine Säuren; dadurch wird eine pH-Verschiebung während der Reaktion vermieden.

Die oben genannten Zuckeralkohole wirken nicht reizend und werden im Lebensmittel- und Kosmetikbereich problemlos eingesetzt. Hinzu kommt ein angenehme Geschmack der Zuckeralkohole, den andere Oxidase-Substrate nicht bieten können, wenn sie nicht sogar unangenehm schmecken oder riechen (z. B. fischig durch Zerfall zu Aminen).

Zuckeralkohole können aus regenerativen Quellen gewönnen werden, was unter ökologischen Gesichtspunkten positiv zu bewerten ist. Viele Zuckeralkohole sind aufgrund ihrer jeweiligen positiven Eigenschaften wie z.B. Kalorienreduktion gegenüber Zuckern, Kariesvorbeugung, oder Verdauungsförderung der Allgemeinheit bereits aus gesundheitsfördernden Produkten bekannt und werden gegenüber anderen Chemikalien bevorzugt.

Zuckeralkohole, besonders z. B. Sorbitol, sind bereits Standard-Formulierungsbestandteile kosmetischer Produkte wie z. B. Zahncreme, wo sie zur Süßung, Feuchthaltung und Einstellung der Fließeigenschaften dienen. Ein Zusatz von Zuckeralkoholoxidasen ist hier also mit einem großen Synergieeffekt in der Formulierung verbunden. Des weiteren wirken Zuckeralkohole stabilisierend auf enzymhallige Formulierungen, was zum einen der Zuckeralkoholoxidase, aber auch evt. weiteren zugegebenen Enzymen eine erhöhte Stabilitat verfeiht, wodurch sich die Möglichkeit ergibt, andere Mehrfachalkohole in Rezepturen durch entsprechende Zuckeralkohole zu ersetzen, z. B. Propylenglycol. Sorbitol ist hierbei im Vergleich zu Xylitol wirtschaftlich günstiger.

Das pH-Profil von in erfindungsgemäßen Mitteln eingesetzten Systemen ist kompatibel mit dem erforderlichen pH bei technischem Einsatz sowie mit typischen Produkten wie Wasch- und Reinigungsmitteln und Haarfärtungen.

In einer bevorzugten Ausführungsform erfindungsgemäßer Mittel befinden sich Polyol-Oxidase und Polyol gemeinsam in einer Zubereitung.

Erfindungsgemäße Mittel können erfindungsgemäß insbesondere in einem Einkammer- oder in einem Mehrkammersystem untergebracht sein, wobei sich Polyol-Oxidase und Polyol in derselben Kammer oder unterschiedlichen Kammern befinden können. In einer bevorzugten Ausführungsform handelt es sich um ein Mehrkammersystem, insbesondere um ein Zweikammersystem, wobei sich Polyot-Oxidase und .Polyol in unterschiedlichen Kammern befinden.

Wird ein erfindungsgemäßes Mittel zur Reinigung bzw. Entfernung von Anschmutzungen verwendet, so kann sich das Polyol gegebenenfalls auch in der zu entfernenden Anschmutzung befinden. Es kann hierbei ausreichend sein, dass sich das Polyol nur in der Anschmutzung befindet. In einer weiteren bevorzugten Ausführungsform eines erfindungsgemäßen Mittels befindet sich daher das Polyol nur in einer zu entfernenden Anschmutzung.

Unter einem Protein ist im Sinne der vorliegenden Anmeldung ein aus den natürlichen Aminosäuren zusammengesetzles, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren sowie Glycin mit den international gebräuchlichen 1- und 3-Buchstaben-Codes bezeichnet.

Unter einem Enzym ist im Sinne der vorliegenden Anmeldung ein Protein zu verstehen, das eine bestimmte biokatalytische Funktion ausübt.

Unter Nukleinsäuren sind im Sinne der vorliegenden Anmeldung die natürlicherweise aus Nukleotiden aufgebauten als Informationsträger diertenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelsirang vorliegen. Als der natürlicherweise dauerhaftere Informationsträger ist die Nukleinsaure DNA für molekularbiologische Arbeiten bevorzugt. Demgegenüber Wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen.

Bei DNA sind die Sequenzen beider komplementärer Stränge in jeweils allen drei möglichen Leserastern zu berücksichtigen. Ferner ist zu berücksichtigen, daß verschiedene Codon-Triplets für dieselben Aminosäuren codieren können, so daß eine bestimmte Aminosäure-Abfolge von mehreren unterschiedlichen und möglicherweise nur geringe Identität aufweisenden Nukleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als Gen bezeichnet.

Die vorliegende Erfindung umfaßt die Herstellung rekombinanter Proteine Hierunter sind erfindungsgemäß alle gentechnischen oder mikrobiologischen Verfahren zu verstehen, die darauf beruhen, daß die Gene für die interessierenden Proteine in einen für die Produktion geeigneten Wirtsorganismus eingebracht und von diesem transkribiert und translatiert werden. Geeigneterweise erfolgt die Einschleusung der betreffenden Gene über Vektoren, insbesondere Expressionsvektoren; aber auch über solche, die bewirken, daß das interessierende Gen im Wirtsorganismus in ein bereits vorhandenes genetisches Element wie das Chromosom oder andere Vektoren eingefügt werden kann. Die funktionelle Einheit aus Gen und Promotor und eventuellen weiteren gegentischen Elementen wird erfindungsgemäß als Expressionskassette bezeichnet. Sie muß dafür jedoch nicht notwendigerweise auch als physische Einheit vorliegen.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press, bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert oder rekombiniert werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions-Substitutionsmutationen oder Fusionen zu deletions-, insertions- substitutionsmutierten oder Fusionsgenen und auf Proteinebene zu entsprechenden Deletions-, Insertions- oder Substitutionsvarianten, beziehungsweise Fusionsproteinen.

Unter Fragmenten werden alle Proteine oder Peptide verstanden, die kleiner sind als natürlichen Proteine oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder katalytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Den Fragmenten entsprechen auf Nukleinsäure-Ebene die Teilsequenzen.

Unter chimären oder hybriden Proteinen sind im Sinne der vorliegenden Anmeldung solche Proteine zu verstehen, die von Nukleinsäureketten kodiert werden, die natürlicherweise von verschiedenen oder aus demselben Organismus stammen. Dieses Vorgehen wird auch Rekombinationsmutagenese genannt. Der Sinn einer solchen Rekombination kann beispielsweise darin bestehen, mithilfe des heranfusionierten Proteinteils eine bestimmte enzymatische Funktion herbeizuführen oder zu modifizieren. Es ist dabei im Sinne der vorliegenden Erfindung unwesentlich, ob solch ein chimäres Protein aus einer einzelnen Polypeptidkette oder mehreren Untereinheiten besteht, auf welche sich unterschiedliche Funktionen verteilen können..

Unter durch Insertionsmutation erhaltene Proteinen sind solche Varianten zu verstehen, die über an sich bekannte Methoden durch Einfügen eines Nukleinsäure-, beziehungsweise Proteinfragments in die Ausgangssequenzen erhalten worden sind. Sie sind ihrer prinzipiellen Gleichartigkeit wegen den chimären Proteinen zuzuordnen. Sie unterscheiden sich von jenen lediglich im Größenverhältnis des unveränderten Proteinteils zur Größe des gesamten Proteins. In solchen insertionsmutierten Proteinen ist der Anteil an Fremdprotein geringer als in chimären Proteinen.

Inversionsmutagenese, also eine partielle Sequenzumkehrung, kann als Sonderform sowohl der Deletion, als auch der Insertion angesehen werden. Dasselbe gilt für eine von der ursprünglichen Aminosäureabfolge abweichende Neugruppierung verschiedener Molekülteile. Sie kann sowohl als Deletionsvariante, als Insertionsvariante, als auch als Shuffling-Variante des ursprünglichen Proteins angesehen werden.

Unter Derivaten werden im Sinne der vorliegenden Anmeldung solche Proteine verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise auch um andere Proteine handeln, die beispielsweise über bifunktionelle chemische Verbindungen an erfindungsgemäße Proteine gebunden werden. Derartige Modifizierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Ebenso ist unter Derivatisierung die kovalente Bindung an einen makromolekularen Träger zu verstehen.

Proteine können auch über die Reaktion mit einem Antiserum oder einem bestimmten Antikörper zu Gruppen immunologisch verwandter Proteine zusammengefaßt werden. Die Angehörigen einer Gruppe zeichnen sich dadurch aus, daß sie dieselbe, von einem Antikörper erkannte antigene Determinante aufweisen.

Im Sinne der vorliegenden Erfindung werden alle Enzyme, Proteine, Fragmente und Derivate, sofern sie nicht explizit als solche angesprochen zu werden brauchen, unter dem Oberbegriff Proteine zusammengefaßt.

Unter Vektoren werden im Sinne der vorliegenden Erfindung aus Nukleinsäuren bestehende Elemente verstanden, die als kennzeichnenden Nukleinsäurebereich ein interessierendes Gen enthalten. Sie vermögen dieses in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfüllen, das interessierende Gen in der Wirtszelle zu realisieren, das heißt, die Expression des betreffenden Proteins zu ermöglichen. Diese Vektoren werden als Expressionsvektoren bezeichnet.

Durch Vergleich mit bekannten Enzymen, die beispielsweise in allgemein zugänglichen Datenbanken hinterlegt sind, läßt sich aus der Aminosäure- oder Nukleotid-Sequenz die enzymatische Aktivität eines betrachteten Enzyms folgern. Diese kann durch andere Bereiche des Proteins, die nicht an der eigentlichen Reaktion beteiligt sind, qualitativ oder quantitativ modifiziert werden. Dies könnte beispielsweise die Enzymstabilität, die Aktivität, die Reaktionsbedingungen oder die Substratspezifität betreffen.

Solch ein Vergleich geschieht dadurch, daß ähnliche Abfolgen in den Nukleotid- oder Aminosäuresequenzen der betrachteten Proteine einander zugeordnet werden. Dies nennt man Homologisierung. Eine tabellarische .Zuordnung der betreffenden Positionen wird als Alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben.

Eine Zusammenstellung aller in den verglichenen Sequenzen übereinstimmenden Positionen wird als Konsensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben Positionen widergegeben. Ein weiter gefaßter Homologiebegriff bezieht die konservierten Aminosäure-Austausche in diesen Wert mit ein. Es ist dann von Prozent Ähnlichkeit die Rede. Solche Aussagen können über ganze Proteine oder Gene oder nur über einzelne Bereiche getroffen werden.

Die Erstellung eines Alignments ist der erste Schritt zur Definition eines Sequenzraums. Dieser hypothetische Raum umfaßt sämtliche durch Permutation in Einzelpositionen abzuleitenden Sequenzen, die sich unter Berücksichtigung aller in den betreffenden Einzelpositionen des Alignments auftretenden Variationen ergeben. Jedes hypothetisch mögliche Proteinmolekül bildet einen Punkt in diesem Sequenzraum. Beispielsweise begründen zwei Aminosäuresequenzen, die bei weitgehender Identität an lediglich zwei verschiedenen Stellen jeweils zwei verschiedene Aminosäuren aufweisen, somit einen Sequenzraum von vier verschiedenen Aminosäuresequenzen. Ein sehr großer Sequenzraum wird erhalten, wenn zu einzelnen Sequenzen eines Raums jeweils weitere homologe Sequenzen gefunden werden. Über solche, jeweils paarweise bestehenden hohen Homologien können auch sehr niedrig homologe Sequenzen als einem Sequenzraum zugehörig erkannt werden.

Homologe Bereiche von verschiedenen Proteinen sind durch Übereinstimmungen in der Aminosäuresequenz definiert. Diese können auch durch identische Funktion gekennzeichnet sein Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes.

Im Rahmen der vorliegenden Erfindung wird die Nukleinsäure geeigneterweise in einen Vektor kloniert. Die molekularbiologische Dimension der Erfindung besteht somit in Vektoren mit den Genen für die entsprechenden Proteine. Dazu können beispielsweise solche gehören, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Die Vektoren bilden geeignete Ausgangspunkte für molekularbiologische und biochemische Untersuchungen des betreffenden Gens oder zugehörigen Proteins und für erfindungsgemäße Weiterentwicklungen und letztlich für die Amplifikation und Produktion erfindungsgemäßer Proteine. Sie stellen insofern Ausführungsformen der vorliegenden Erfindung dar, als die Sequenzen der enthaltenen erfindungsgemäß einsetzbaren Nukleinsäurebereiche jeweils innerhalb der oben näher bezeichneten Homologiebereiche liegen.

Klonierungsvektoren eignen sich neben der Lagerung, der biologischen Amplifikation oder der Selektion des interessierenden Gens für die Charakterisierung des betreffenden Gens, etwa über das Erstellen einer Restriktionskarte oder die Sequenzierung.

Expressionsvektoren sind chemisch den Klonierungsvektoren ähnlich, unterscheiden sich aber in jenen Teilsequenzen, die sie dazu befähigen, in den für die Produktion von Proteinen optimierten Wirtsorganismen zu replizieren und dort das enthaltene Gen zur Expression zu bringen. Die Expression wird beispielsweise von Promotoren beeinflußt, welche die Transkription des Gens regulieren. So kann die Expression durch den natürlichen, ursprünglich vor diesem Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus.

Bevorzugt sind solche Expressionsvektoren, die über Änderungen der Kulturbedingungen oder Zugabe von bestimmten Verbindungen, wie beispielsweise die Zelldichte oder spezielle Faktoren, regulierbar sind. Expressionsvektoren ermöglichen, daß das zugehörige Protein heterolog, also in einem anderen Organismus als dem, aus dem es natürlicherweise gewonnen werden kann, produziert wird. Auch eine homologe Proteingewinnung aus einem das Gen natürlicherweise exprimierenden Wirtsorganismus über einen passenden Vektor liegt innerhalb des Schutzbereichs der vorliegenden Erfindung. Diese kann den Vorteil aufweisen, daß natürliche, mit der Translation in einem Zusammenhang stehende Modifikationsreaktionen an dem entstehenden Protein genauso durchgeführt werden, wie sie auch natürlicherweise ablaufen würden.

Erfindungsgemäß einsetzbar können auch zellfreie Expressionssysteme sein, bei denen die Proteinbiosynthese in vitro nachvollzogen wird. Derartige Expressionssysteme sind im Stand der Technik ebenfalls etabliert.

Die In-vivo-Synthese eines Enzyms, also die durch lebende Zellen, erfordert den Transfer des zugehörigen Gens in eine Wirtszelle, deren sogenannte Transformation. Als Wirtszellen eignen sich prinzipiell alle Organismen, das heißt Prokaryonten oder Eukaryonten. Bevorzugt sind solche Wirtszellen, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Protein kann auf diese Weise aus einer Vielzahl von Wirtsorganismen gewonnen werden.

Bevorzugt sind solche Wirtszellen, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine sehr wirtschaftliche Produktion der interessierenden Proteine.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung der interessierenden Proteine etabliert werden. Bei gram-negativen Bakterien, wie beispielsweise Escherichia coli (E. coli), werden eine Vielzahl von Proteinen in den periplasmatischen Raum sekretiert, also in das Kompartiment zwischen den beiden die Zellen einschließenden Membranen. Dies kann für spezielle Anwendungen vorteilhaft sein. Grampositive Bakterien, wie beispielsweise Bacilli oder Actinomyceten oder andere Vertreter der Actinomycetales, besitzen demgegenüber keine äußere Membran, so daß sekretierte Proteine sogleich in das die Zellen umgebende Nährmedium abgegeben werden, aus welchem sich nach einer anderen bevorzugten Ausführungsform die exprimierten erfindungsgemäß einsetzbaren Proteine direkt aufreinigen lassen.

Eine Variante dieses Produktionsprinzips stellen Expressionssysteme dar, bei denen zusätzliche Gene, beispielsweise solche, die auf anderen Vektoren zur Verfügung gestellt werden, die Produktion interessierender Proteine beeinflussen. Hierbei kann es sich um modifizierende Genprodukte handeln oder um solche, die mit dem interessierenden Protein gemeinsam aufgereinigt werden sollen, etwa um dessen enzymatische Funktion zu beeinflussen. Dabei kann es sich beispielsweise um andere Proteine oder Enzyme, um Inhibitoren oder um solche Elemente handeln, die die Wechselwirkung mit verschiedenen Substraten beinflussen.

Aufgrund der weitreichenden Erfahrungen, die man beispielsweise hinsichtlich der molekularbiologischen Methoden und der Kultivierbarkeit mit coliformen Bakterien hat, sind diese zur Gewinnung erfindungsgemäß einsetzbarer Enzyme bevorzugt. Besonders bevorzugt sind solche der Gattungen Escherichia coli, insbesondere nichtpathogene, für die biotechnologische Produktion geeignete Stämme.

Repräsentative Vertreter dieser Gattungen sind die K12-Derivate und die B-Stämme von Escherichia coli. Stämme, die sich nach an sich bekannten genetischen und/oder mikrobiologischen Methoden von diesen ableiten lassen, und somit als deren Derivate angesehen werden können, besitzen für genetische und mikrobiologische Arbeiten die größte Bedeutung und werden vorzugsweise zur Entwicklung erfindungsgemäßer Verfahren eingesetzt. Solche Derivate können beispielsweise über Deletions- oder Insertionsmutagenese hinsichtlich ihrer Anforderungen an die Kulturbedingungen verändert sein, andere oder zusätzliche Selektionsmarker aufweisen oder andere oder zusätzliche Proteine exprimieren. Es kann sich insbesondere um solche Derivate handeln, die zusätzlich zu dem erfindungsgemäß hergestellten Protein weitere wirtschaftlich interessante Proteine exprimieren.

Dem Fachmann ist wohlbekannt, daß die heterologe Expression von Genen besonders dann gelingt, wenn die gewählte Wirtszelle dem Organismus, aus dem das Ursprungsgen stammt, besonders nahe verwandt ist. Deshalb sind auch Expressionssysteme innerhalb der Actinomyceten bevorzugte Systeme zur Expression, bevorzugt werden besonders Streptomyceten, darunter Streptomyces coelicolor, Streptomyces avermitilis, Streptomyces lividans und prinzipiell besonders solche Streptomyceten-Stämme, die sich durch entsprechende Auswahl oder genetische Manipulation besonders für eine technische Produktion von Enzymen eignen.

Auch solche Mikroorganismen sind bevorzugt, die dadurch gekennzeichnet sind, daß sie nach Transformation mit einem der oben beschriebenen Vektoren erhalten worden sind. Dabei kann es sich beispielsweise um Klonierungsvektoren handeln, die zur Lagerung und/oder Modifikation in einen beliebigen Bakterienstamm eingebracht worden sind. Solche Schritte sind in der Lagerung und in der Weiterentwicklung betreffender genetischer Elemente allgemein verbreitet. Da aus diesen Mikroorganismen die betreffenden genetischen Elemente in zur Expression geeignete gram-negative Bakterien unmittelbar übertragen werden können, handelt es sich auch bei den vorangegenagenen Transformationsprodukten um Verwirklichungen des betreffenden Erfindungsgegenstands.

Auch eukaryontische Zellen können sich zur Produktion interessierender Proteine eignen. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccaride. '

Die Wirtszellen werden in an sich bekannter Weise kultiviert und fermentiert, beispielsweise in diskontinuierlichen oder kontinuierlichen Systemen. Im ersten Fall wird ein geeignetes Nährmedium mit den Organismen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig Produkt aus dem Medium entnommen werden kann.

Fermentationsverfahren sind an sich aus dem Stand der Technik wohlbekannt und stellen den eigentlichen großtechnischen Produktionsschritt dar; gefolgt von einer geeigneten Aufreinigungsmethode.

Alle Fermentationsverfahren, die auf einem der oben ausgeführten Verfahren zur Herstellung der rekombinanten Proteine beruhen, stellen entsprechend bevorzugte Ausführungsformen dieses Erfindungsgegenstandes dar.

Hierbei müssen die für die eingesetzten Herstellungsverfahren, für die Wirtszellen und/oder die herzustellenden Proteine jeweils optimalen Bedingungen anhand der zuvor optimierten Kulturbedingungen der betreffenden Stämme nach dem Wissen des Fachmanns, beispielsweise hinsichlich Fermentationsvolumen, Medienzusammensetzung, Sauerstoffversorgung oder Rührergeschwindigkeit experimentell ermittelt werden.

Fermentationsverfahren, die dadurch gekennzeichnet sind, daß die Fermentation über eine Zulaufstrategie durchgeführt wird, kommen ebenfalls in Betracht. Hierbei werden, die Medienbestandteile, die durch die fortlaufende Kultivierung verbraucht werden, zugefüttert; man spricht auch von einer Zufütterungsstrategie. Hierdurch können beträchtliche Steigerungen sowohl in der Zelldichte, als auch in der Biotrockenmasse und/oder vor allem der Aktivität des interessierenden Proteins erreicht werden.

Analog dazu kann die Fermentation auch so gestaltet werden, daß unerwünschte Stoffwechselprodukte herausgefiltert oder durch Zugabe von Puffer oder jeweils passende Gegenionen neutralisiert werden.

Das hergestellte Protein kann nachträglich aus dem Fermentationsmedium geerntet werden. Dieses Fermentationsverfahren ist gegenüber der Produktaufbereitung aus der Trockenmasse bevorzugt, erfodert jedoch die Zurverfügungstellung geeigneter Sekretionsmarker und Transportsysteme.

Ohne Sekretion ist u.U. die Aufreinigung des Proteins aus der Zellmasse erforderlich, auch dazu sind verschiedene Verfahrer bekannt, wie Fällung z.B durch Ammoniumsulfat oder Ethanol, oder die chromatographische Reinigung, wenn erforderlich bis zur Homogenität. Die Mehrzahl der beschriebenen technischen Verfahren dürfte jedoch mit einer angereicherten, stabilisierten Präparation auskommen.

Alle bereits oben ausgeführten Elemente können zu Verfahren kombiniert werden, um interessierende Proteine herzustellen. Es ist dabei für jedes interessierende Protein eine Vielzahl von Kombinationsmöglichkeiten an Verfahrensschritten denkbar. Das optimale Verfahren, muß für jeden konkreten Einzelfall experimentell ermittelt werden.

Durch Expression oder Klonierung können die interessierenden Oxidasen in der für den technischen Einsatz erforderlichen Menge zur Verfügung gestellt werden.

Die erfindungsgemäß einsetzbaren Polyol-Oxidasen weisen ein pH-Optimum vorzugsweise im schwach sauren bis alkalischen Bereich von etwa pH 4 bis pH 12, insbesondere pH 6 bis pH 11, bevorzugt pH 8 bis pH 10 und besonders bevorzugt von etwa pH 10 auf.

Die Aktivität solcher Enzyme wird üblicherweise in U ausgedrückt, wobei die Einheit ("Unit") derjenigen Enzymmenge entspricht, die 1 µmol an Wasserstoffperoxid (H₂O₂) bei einem festgelegten pH und einer festgelegten Temperatur in 1 Minute generiert.

Das Temperatur-Optimum der erfindungsgemäß einsetzbaren Polyol-Oxidasen liegt etwa im Bereich von 20 bis 60°C, insbesondere im Bereich von 30 bis 60°C und besonders bevorzugt bei etwa 50°C.

Eine erfindungsgemäße Polyoloxidase weist beispielsweise bezogen auf das jeweilige Substrat eine spezifische Aktivität auf, die aus den in der folgenden Tabelle aufgeführten spezifischen Aktivitäten ausgewählt ist:

| | vorzugsweise | insbesondere | besonders bevorzugt | ganz besonders bevorzugt |
|---|---|---|---|---|
| D-Sorbitol | 0,1-500 | 0,5-200 | 1-80 | 5-10 |
| Xylitol | 0,1-500 | 0,5-200 | 1-80 | 5-10 |
| D-Mannitol | 0,1-450 | 05-90 | 1-40 | 5-10 |
| D-Arabitol | 0,1-450 | 0,5-90 | 1-40 | 5-10 |
| 1,4-Butandiol | 0,1-450 | 0,5-90 | 1-40 | 5-10 |
| Glycerol | 0,1-400 | 0,5-150 | 1-80 | 5-10 |
| Polyvinylalkohol | 0,1-500 | 0,5-150 | 1-80 | 5-10 |
| Erythritol | 0,1-400 | 0,5-80 | 1-40 | 5-10 |
| Adonitol | 0,1-350 | 0,3-80 | 1-40 | 5-10 |
| Ethylenglycol | 0.1-500 | 0,5-200 | 1-100 | 5-10 |

Die Menge des im erfindungsgemäßen Mittel, insbesondere Waschmittel, enthaltenen Substrats für die Oxidase richtet sich nach der zum Erzielen des gewünschten Bleichergebnisses erforderlichen Wasserstoffperoxidmenge. Hier kann als Anhaltspunkt dienen, daß bei Enzym-Substrat-Systemen, die pro Mol umgesetztem Substrat ein Mol Wasserstoffperoxid freisetzen, die Anwesenheit von etwa 0,05 Gew.% bis 1 Gew.% des Substrats in der Wasch-, Bleich- oder Reinigungsflotte in der Regel zur Erzielung eines guten Bleichergebnisses genügt. Falls sich bereits in der Anschmutzung genügend Substrat befindet, kann, wie bereils zuvor ausgeführt, gegebenenfalls auch auf die Zugabe von Substrat verzichlet werden.

Zur Klonierung der erfingdungsgemäß einsetzbaren Polyol-Oxidase wurden neue PCR-Primer (Seq. 6 und 7) konstruiert.

Weitere Gegenstände der vorliegenden Erfindung sind somit Oligonukleolide, insbesondere PCR-Primer, mit einer der in Seq. 6 und 7 angegebenen Sequenzen, sowie Oligonukleotide, deren Nukleotidsequenzen mit einer der in Seq. 6 und 7 angegebenen Nukleolidsequenzen zu mindestens 85 %, insbesondere mindestens 90 %, besonders bevorzugt mindestens 95 % und ganz besonders bevorzugt zu 100 % übereinstimmen..

Die erfindungsgemäß einsetzbaren Oligonukleolide oder Nukleinsäuren sind zur Identifizierung und/oder Gewinnung einer neuen Polyol-Oxidase verwendbar.

Die erfindungsgemaß einsetzbaren Polyol-Oxidasen können vorteilhaft in Körperpflegemittel, Haarpflegemittel. Haarwaschmittel; Süßigkeiten, insbesondere zahnpflegende Süßigkeiten. z. B. Bonbons oder Kaugummis; Mund-, Zahn- und Zahnprothesenpflegemittel, Zahnspangenpflegemittel, Kosmetika, Therapeutika, Waschmittel, Reinigungsmitlel, Aufhellungsmittel, Bleichmittel, Desinfektionsmittel, Nachspülmittel, Handwaschmittel, Handgeschirrspülmittel, Maschinengeschirrspülmiltel und Mittel zur bleichenden oder desinfizierenden Behandlung von Filtermedien, Textilien, Pelzen, Papier, Fellen oder Leder eingebracht werden.

Erfindungsgemäß bevorzugt ist ein Wasch-, oder Bleichmittel, enthaltend ein Bleichsystem, das in der Lage ist, unter Anwendungsbedingungen des Mittels Wasserstoffperoxid zu erzeugen, und gegebenenfalls synthetisches Tensid, organischen und/oder anorganischen Builder sowie sonstige übliche Bleich- oder Waschmittelbestandteile, dadurch gekennzeichnet, daß das Bleichsystem aus einer erfindungsgemäß einsetzbaren Polyol-Oxidase und einem Substrat für die Polyol-Oxidase besteht. Das Substrat ist vorzugsweise Sorbitol, Xylitol, Glycerol, Ethylenglycol oder Polyvinylalkohol oder ein anderes bevorzugtes Substrat, wie oben beschrieben.

Das erfindungsgemäße Wasch-, oder Bleichmittel weist vorzugsweise eine Polyoloxidase-Aktivität von 1 U/g bis 2000 U/mg auf; es liegt bevorzugtermaßen als rieselfähiges Pulver mit einem Schüttgewicht von 300 g/l bis 1200 g/l, insbesondere 500 g/l bis 900 g/l, vor. Alternativ kann es aber auch in Form eines pastenförmigen oder flüssigen Waschmittels vorliegen, insbesondere in Form eines nicht-wäßrigen Flüssigwaschmittels oder einer nicht-wäßrigen Paste oder in Form eines wäßrigen Flüssigwaschmittels oder einer wasserhaltigen Paste.

Das erfindungsgemäße Wasch-, oder Bleichmittel kann in einem luftundurchlässigen Behältnis verpackt sein, aus dem es kurz vor Gebrauch oder während des Waschvorgangs freigesetzt wird, insbesondere kann die Polyol-Oxidase und/oder das Substrat für dieses Enzym mit einer bei Raumtemperatur oder bei Abwesenheit von Wasser für das Enzym und/oder dessen Substrat undurchlässigen Substanz umhüllt sein, welche unter Anwendungsbedingungen des Mittels durchlässig für das Enzym und/oder dessen Substrat wird.

Das erfindungsgemäße Wasch-, oder Bleichmittel enthält vorzugsweise zusätzlich zum Bleichsystem
- 5 Gew.% bis 70 Gew.%, insbesondere 10 Gew.-% bis 50 Gew.% Tensid,
- 10 Gew.% bis 65 Gew.-%, insbesondere 12 Gew.-% bis 60 Gew. -% wasserlösliches, wasserdispergierbares anorganisches Buildermaterial,
- 1 Gew.-% bis 10 Gew.-%, insbesondere 2 Gew.-% bis 8 Gew.-%, wasserlösliche organische Buildersubstanzen,
- nicht über 15 Gew.% feste anorganische und/oder organische Säuren beziehungsweise saure Salze,
- nicht über 5 Gew.% Komplexbildner für Schwermetalle,
- nicht über 5 Gew.% Vergrauungsinhibitor,
- nicht über 5 Gew. % Farbübertragungsinhibitor und
- nicht über 5 Gew.-% Schauminhibitor.

Das erfindungsgemäße Mittel kann auch in einem Mehrkammersystem, insbesondere Zweikammersystem, untergebracht sein, wobei sich in einer bevorzugten Ausführungsform Polyol-Oxidase und Substrat in zwei unterschiedlichen Kammern befinden, um die vorzeitige Erzeugung von Wasserstoffperoxid zu unterbinden.

Weiterer bevorzugter Gegenstand der vorliegenden Erfindung ist ein Verfahren zur Entfernung einer Anschmutzung, dadurch gekennzeichnet, dass eine Polyol-Oxidase, insbesondere eine Zuckeralkoholoxidase, vorzugsweise eine solche, deren Aminosäuresequenz mit der in Seq. 4 angegebenen Aminosäuresequenz zu mindestens 85 %, insbesondere mindestens 90 %, besonders bevorzugt mindestens 95 % und ganz besonders bevorzugt zu 100 % übereinstimmt, auf die zu entfernende Anschmutzung aufgebracht wird.

Hierbei wird in einer bevorzugten Ausführungsform gleichzeitig oder zeitlich versetzt ein Substrat der Zuckeralkoholoxidase auf die Anschmutzung aufgebracht, wobei sich Zuckeralkoholoxidase und Substrat der Zuckeralkoholoxidase in unterschiedlichen Kammern eines Mehrkammersystems befinden können.

In einer weiteren bevorzugten Ausführungsform des erfindungsgemäßen Verfahrens befindet sich das Substrat der Zuckeralkoholoxidase bereits in der Anschmutzung, so dass auf die Zugabe von zusätzlichem Substrat gegebenenfalls auch verzichtet werden kann.

Aufgrund ihrer großen technischen Bedeutung werden nun in Ergänzung zu den bisher dargestelliten besonders bevorzugten Ausführungsformen detailliert die verschiedenen Aspekte und sonstigen Inhaltsstoffe erfindungsgemäßer, d.h. durch die oben beschriebenen Polyol-Oxidasen gekennzeichneter Wasch- und Reinigungsmittel beschrieben.

Hierbei wird global nach dem Waschgut zwischen Textilien und festen Oberflächen unterschieden. Die hierfür zu wählenden, insbesondere über die sonstigen Inhaltsstoffe zu steuernden Bedingungen, wie beispielsweise Temperatur, pH-Wert, lonenstärke, Redox-Verhältnisse oder mechanische Einflüsse sollten für das jeweilige Reinigungsproblem optimiert sein. So liegen übliche Temperaturen für Wasch- und Reinigungsmittel in Bereichen von 10°C bei manuellen Mitteln über 40°C und 60°C bis hin zu 95° bei maschinellen Mitteln oder bei technischen Anwendungen. Da bei modernen Wasch- und Spülmaschinen die Temperatur meist stufenlos einstellbar ist, sind auch alle Zwischenstufen der Temperatur eingeschlossen. Vorzugsweise werden die Inhaltsstoffe der betreffenden Mittel aufeinander abgestimmt. Bevorzugt sind Synergien hinsichtlich der Reinigungsleistung.

Eine erfindungsgemäße Polyol-Oxidase kann sowohl in Mitteln für Großverbraucher oder technische Anwender als auch in Produkten für den Privatverbraucher Anwendung finden, wobei alle im Stand der Technik etablierten Reinigungsmittelarten auch Ausführungsformen der vorliegenden Erfindung darstellen. Dazu gehören beispielsweise Konzentrate und unverdünnt anzuwendende Mittel; zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet.

Ausführungsformen der vorliegenden Erfindung umfassen alle etablierten und/oder alle zweckmäßigen Darreichungsformen. Dazu zählen beispielsweise feste, pulverförmige, flüssige, gelförmige oder pastöse Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt.

Die erfindungsgemäß einsetzbaren Polyol-Oxidasen werden in erfindungsgemäßen Mitteln beispielsweise mit einzelnen oder mehreren der folgenden Inhaltsstoffe kombiniert: nichtionische, anionische und/oder kationische Tenside, (gegebenenfalls weitere) Bleichmittel, Bleichaktivatoren, Bleichkatalysatoren, Builder und/oder Cobuilder, Lösungsmittel, Verdicker, Sequestrierungsmittel, Elektrolyte, optische Aufheller, Vergrauungsinhibitoren, Korrosionsinhibitoren, insbesondere Silberschutzmittel, Soil-Release-Wirkstoffe, Farbtransfer(oder -Übertragungs) -Inhibitoren, Schauminhibitoren, Abrasivstoffe, Farbstoffe, Duftstoffe, antimikrobielle Wirkstoffe, UV-Schutzmittel, Enzyme wie beispielsweise Proteasen, Amylasen, Lipasen, Cellulasen, Hemicellulasen oder Oxidasen, Stabilisatoren, insbesondere Enzymstabilisatoren, und andere Komponenten, die aus dem Stand der Technik bekannt sind.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann, beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhafterweise eingesetzt werden können, sind die Alkylpolyglycoside (APG). Einsetzbare Alkypolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosylierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Der Anteil dieser nichtionischen Tenside liegt vorzugsweise nicht über dem der ethoxylierten Fettalkohole, insbesondere bei nicht mehr als der Hälfte davon.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen bis 5 Gew.%, üblicherweise von 1 bis 5 Gew.%, eingesetzt.

Weitere geeignete Aniontenside sind auch die Salze der Alkylsulfobemsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, zum Beispiel Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Die Tenside können in den erfindungsgemäßen Reinigungs- oder Waschmitteln insgesamt in einer Menge von vorzugsweise 5 Gew.% bis 50 Gew.%, insbesondere von 8 Gew.% bis 30 Gew.%, bezogen auf das fertige Mittel, enthalten sein.

Erfindungsgemäße Mittel können weitere Bleichmittel enthalten. Unter den als Bleichmittel dienenden, in Wasser H₂₀O₂ liefernden Verbindungen haben das Natriumpercarbonat, das Natriumperborattetrahydrat und das Natriumperboratmonohydrat besondere Bedeutung. Weitere brauchbare Bleichmittel sind beispielsweise Peroxopyrophosphate, Citratperhydrate sowie H₂O₂ liefernde persaure Salze oder Persäuren, wie Persulfate beziehungsweise Perschwefelsäure. Brauchbar ist auch das Harnstoffperoxohydrat Percarbamid, das durch die Formel H₂N-CO-NH_{2·}H₂O₂ beschrieben werden kann. Insbesondere beim Einsatz der Mittel für das Reinigen harter Oberflächen, zum Beispiel beim maschinellen Geschirrspülen, können sie gewünschtenfalls auch Bleichmittel aus der Gruppe der organischen Bleichmittel enthalten, obwohl deren Einsatz prinzipiell auch bei Mitteln für die Textilwäsche möglich ist. Typische organische Bleichmittel sind die Diacylperoxide, wie zum Beispiel Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind die Peroxybenzoesäure und ihre ringsubstituierten Derivate, wie Alkylperoxybenzoesäuren, aber auch Peroxy-α-Naphthoesäure und Magnesium-monoperphthalat, die aliphatischen oder substituiert aliphatischen Peroxysäuren, wie Peroxylaurinsäure, Peroxystearinsäure, ε-Phthalimidoperoxycapronsäure (Phthalimidoperoxyhexansäure, PAP), o-Carboxybenzamidoperoxycapronsäure, N-Nonenylamidoperadipinsäure und N-Nonenylamidopersuccinate, und aliphatische und araliphatische Peroxydicarbonsäuren, wie 1,12-Diperoxycarbonsäure, 1,9-Diperoxyazelainsäure, Diperoxysebacinsäure, Diperoxybrassylsäure, die Diperoxyphthalsäuren, 2-Decyldiperoxybutan-1,4-disäure, N,N-Terephthaloyl-di(6-aminopercapronsäure) können eingesetzt werden.

Der Gehalt der Mittel an Bleichmittel kann 1 bis 40 Gew.% und insbesondere 10 bis 20 Gew.-%, betragen, wobei vorteilhafterweise Perboratmonohydrat oder Percarbonat eingesetzt wird. Eine synergistische Verwendung von Amylase mit Percarbonat oder von Amylase mit Percarbonsäure wird mit den Anmeldungen WO 99/63036, beziehungsweise WO 99/63037 offenbart.

Um beim Waschen bei Temperaturen von 60°C und darunter, und insbesondere bei der Wäschevorbehandlung eine verbesserte Bleichwirkung zu erreichen, können die Mittel auch Bleichaktivatoren enthalten. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glycolurile, insbesondere 1,3,4,6-Tetraacetylglycoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- beziehungsweise iso-NOBS), acylierte Hydroxycarbonsäuren, wie Triethyl-O-acetylcitrat (TEOC), Carbonsäureanhydride, insbesondere Phthalsäureanhydrid, Isatosäureanhydrid und/oder Bernsteinsäureanhydrid, Carbonsäureamide, wie N-Methyldiacetamid, Glycolid, acylierte mehrwertige Alkohole, insbesondere Triacetin, Ethylenglycoldiacetat, Isopropenylacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglucose (PAG), Pentaacetylfructose, Tetraacetylxylose und Octaacetyllactose sowie acetyliertes, gegebenenfalls N-alkyliertes Glucamin beziehungsweise Gluconolacton, Triazol beziehungsweise Triazolderivate und/oder teilchenförmige Caprolactame und/oder Caprolactamderivate, bevorzugt N-acylierte Lactame, beispielsweise N-Benzoylcaprolactam und N-Acetylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 44 43 177 bekannten Kombinationen konventioneller Bleichaktivatoren können eingesetzt werden. Ebenso können Nitrilderivate wie Cyanopyridine, Nitrilquats, zum Beispiel N-Alkylammoniumacetonitrile, und/oder. Cyanamidderivate eingesetzt werden. Bevorzugte Bleichaktivatoren sind Natrium-4-(octanoyloxy)-benzolsulfonat, n-Nonanoyl- oder Isononanoyloxybenzolsulfonat (n- beziehungsweise iso-NOBS), Undecenoyloxybenzolsulfonat (UDOBS), Natriumdodecanoyloxybenzolsulfonat (DOBS), Decanoyloxybenzoesäure (DOBA, OBC 10) und/oder Dodecanoyloxybenzolsulfonat (OBS 12), sowie N-Methylmorpholinum-acetonitril (MMA). Derartige Bleichaktivatoren können im üblichen Mengenbereich von 0,01 bis 20 Gew.-%, vorzugsweise in Mengen von 0,1 bis 15 Gew.%, insbesondere 1 Gew.% bis 10 Gew.%, bezogen auf die gesamte Zusammensetzung, enthalten sein.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze beziehungsweise Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V-und Cu-Komplexe mit N-haltigen Tripod-Liganden sowie Co-, Fe-, Cu- und Ru-Aminkomplexe sind als Bleichkatalysatoren geeignet, wobei solche Verbindungen bevorzugt eingesetzt werden, die in der DE 197 09 284 A1 beschrieben sind. Gemäß WO 99/63038 vermögen auch Acetonitril-Derivate und gemäß WO 99/63041 bleichaktivierende Übergangsmetallkomplexverbindungen in Kombination mit Amylasen eine bleichaktivierende Wirkung zu entfalten.

Als Bleichaktivator können auch Enzyme eingesetzt werden, die mit Wasserstoffperoxid eine Perhydrolysereaktion eines Substrates katalysieren, so daß Persäuren entstehen. Solche Enzyme können sich ableiten von Lipasen oder Cutinasen, oder auch von Esterasen oder Proteasen, wenn dort nämlich ganz oder teilweise eine Perhydrolyse-Reaktion statt einer Hydrolyse stattfindet. Solche Perhydrolasen sind z.B. in WO2005/056782 (auf der Basis einer Esterase) oder WO2004/058961 (auf der Basis eines modifizierten Subtilisins Carlsberg) beschrieben. Die Verwendung des erfindungsgemäßen Bleichsystems unter Zuhilfenahme von Perhydrolasen ist auch Bestandteil der vorliegenden Erfindung. Erfindungsgemäße Mittel können somit Lipasen oder Cutinasen enthalten, einerseits wegen ihrer Triglycerid-spaltenden Aktivitäten, aber auch, um aus geeigneten Vorstufen *in situ* Persäuren zu erzeugen. Hierzu gehören beispielsweise die ursprünglich aus *Humicola lanuginosa (Thermomyces lanuginosus)* erhältlichen, beziehungsweise weiterentwickelten Lipasen, insbesondere solche mit dem Aminosäureaustausch D96L. Sie werden beispielsweise von der Firma Novozymes unter den Handelsnamen Lipolase^{®}, Lipolase^{®}Ultra, LipoPrime^{®}, Lipozyme^{®} und Lipex^{®} vertrieben. Desweiteren sind beispielsweise die Cutinasen einsetzbar, die ursprünglich aus *Fusarium solani pisi* und *Humicola insolens* isoliert worden sind. Ebenso brauchbare Lipasen sind von der Firma Amano unter den Bezeichnungen Lipase CE^{®}, Lipase P^{®}, Lipase B^{®}, beziehungsweise Lipase CES^{®}, Lipase AKG^{®}, Bacillis sp. Lipase^{®}, Lipase AP^{®}, Lipase M-AP^{®} und Lipase AML^{®} erhältlich. Von der Firma Genencor sind beispielsweise die Lipasen, beziehungsweise Cutinasen einsetzbar, deren Ausgangsenzyme ursprünglich aus *Pseudomonas mendocina* und *Fusarium solanii* isoliert worden sind. Als weitere wichtige Handelsprodukte sind die ursprünglich von der Firma Gist-Brocades vertriebenen Präparationen M1 Lipase^{®} und Lipomax^{®} und die von der Firma Meito Sangyo KK, Japan, unter den Namen Lipase MY-30^{®}, Lipase OF^{®} und Lipase PL^{®} vertriebenen Enzyme zu erwähnen, ferner das Produkt Lumafast^{®} von der Firma Genencor.

Erfindungsgemäße Mittel enthalten in der Regel einen oder mehrere Builder, insbesondere Zeolithe, Silikate, Carbonate, organische Cobuilder und - wo keine ökologischen Gründe gegen ihren Einsatz sprechen - auch die Phosphate. Letztere sind insbesondere in Reinigungsmitteln für das maschinelle Geschirrspülen bevorzugt einzusetzende Gerüststoffe.

Zu nennen sind hier kristalline, schichtförmige Natriumsilikate der allgemeinen Formel NaMSiₓO₂ₓ₊₁·yH₂O, wobei M Natrium oder Wasserstoff bedeutet, x eine Zahl von 1,6 bis 4, vorzugsweise 1,9 bis 4,0 und y eine Zahl von 0 bis 20 ist und bevorzugte Werte für x 2, 3 oder 4 sind. Derartige kristalline Schichtsilicate werden beispielsweise in der europäischen Patentanmeldung EP 0 164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅.yH₂O bevorzugt. Im Handel befinden sich derartige Verbindungen beispielsweise unter der Bezeichnung SKS^{®} (Fa. Clariant). So handelt es sich bei SKS-6^{®} vorwiegend um ein δ-Natriumdisilicat mit der Formel Na₂Si₂O₅₋yH₂O, bei SKS-7^{®} vorwiegend um das β-Natriumdisilicat. Durch Reaktion mit Säuren (zum Beispiel Citronensäure oder Kohlensäure) entsteht aus dem δ-Natriumdisilicat Kanemit NaHSi₂O_{5·}yH₂O, im Handel unter den Bezeichnungen SKS-9^{®} beziehungsweise SKS-10^{®} (Fa. Clariant). Von Vorteil kann es auch sein, chemische Modifikationen dieser Schichtsilicate einzusetzen. So kann beispielsweise die Alkalität der Schichtsilicate geeignete beeinflußt werden. Mit Phosphat beziehungsweise mit Carbonat dotierte Schichtsilicate weisen im Vergleich zu dem δ-Natriumdisilicat veränderte Kristallmorphologien auf, lösen sich schneller und zeigen im Vergleich zu δ-Natriumdisilicat ein erhöhtes Calciumbindevermögen. So sind Schichtsilicate der allgemeinen Summenformel x Na₂ O· y SiO₂ · z P₂O₅, in der das Verhältnis x zu y einer Zahl 0,35 bis 0,6, das Verhältnis x zu z einer Zahl von 1,75 bis 1200 und das Verhältnis y zu z einer Zahl von 4 bis 2800 entsprechen, in der Patentanmeldung DE 196 01 063 beschrieben. Die Löslichkeit der Schichtsilicate kann auch erhöht werden, indem besonders feinteilige Schichtsilicate eingesetzt werden. Auch Compounds aus den kristallinen Schichtsilicaten mit anderen Inhaltsstoffen können eingesetzt werden. Dabei sind insbesondere Compounds mit Cellulosederivaten, die Vorteile in der desintegrierenden Wirkung aufweisen und insbesondere in Waschmitteltabletten eingesetzt werden, sowie Compounds mit Polycarboxylaten, zum Beispiel Citronensäure, beziehungsweise polymeren Polycarboxylaten, zum Beispiel Copolymeren der Acrylsäure, zu nennen.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche löseverzögert sind und Sekundarwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/ Verdichtung oder durch Übertrocknung hervorgerufen worden sein. Im Rahmen dieser Erfindung wird unter dem Begriff "amorph" auch "röntgenamorph" verstanden. Dies heißt, daß die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führten, wenn die Silikatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silikate, compoundierte amorphe Silikate und übertrocknete röntgenamorphe Silikate.

Ein gegebenenfalls einsetzbarer, feinkristalliner, synthetischer und gebundenes Wasser enthaltender Zeolith ist vorzugsweise Zeolith A und/oder P. Als Zeolith P wird Zeolith MAP^{®} (Handelsprodukt der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.% Zeolith X), das von der Firma CONDEA Augusta S.p.A. unter dem Markennamen VEGOBOND AX vertrieben wird und durch die Formel

nNa₂O * (1-n)K₂O Al₂O₃ ·(2 - 2,5)SiO₂· (3,5- 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist auch ein Einsatz der allgemein bekannten Phosphate als Buildersubstanzen möglich, sofern ein derartiger Einsatz nicht aus ökologischen Gründen vermieden werden sollte. Unter der Vielzahl der kommerziell erhältlichen Phosphate haben die Alkalimetallphosphate unter besonderer Bevorzugung von Pentanatrium- beziehungsweise Pentakaliumtriphosphat (Natriumbeziehungsweise Kaliumtripolyphosphat) in der Wasch- und Reinigungsmittel-Industrie die größte Bedeutung.

Alkalimetallphosphate ist dabei die summarische Bezeichnung für die Alkalimetall- (insbesondere Natrium- und Kalium-) -Salze der verschiedenen Phosphorsäuren, bei denen man Metaphosphorsäuren (HPO₃), und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen beziehungsweise Kalkinkrustationen in Geweben und tragen überdies zur Reinigungsleistung bei.

Natriumdihydrogenphosphat, NaH₂PO₄, existiert als Dihydrat (Dichte 1,91 gcm⁻³, Schmelzpunkt 60°) und als Monohydrat (Dichte 2,04 gcm⁻³). Beide Salze sind weiße, in Wasser sehr leicht lösliche Pulver, die beim Erhitzen das Kristallwasser verlieren und bei 200°C in das schwach saure Diphosphat (Dinatriumhydrogendiphosphat, Na₂H₂P₂O₇), bei höherer Temperatur in Natiumtrimetaphosphat (Na₃P₃O₉) und Maddrellsches Salz (siehe unten), übergehen. NaH₂PO₄ reagiert sauer; es entsteht, wenn Phosphorsäure mit Natronlauge auf einen pH-Wert von 4,5 eingestellt und die Maische versprüht wird. Kaliumdihydrogenphosphat (primäres oder einbasiges Kaliumphosphat, Kaliumbiphosphat, KDP), KH₂PO₄, ist ein weißes Salz der Dichte 2,33 gcm⁻³, hat einen Schmelzpunkt 253° [Zersetzung unter Bildung von Kaliumpolyphosphat (KPO₃)ₓ] und ist leicht löslich in Wasser.

Dinatriumhydrogenphosphat (sekundäres Natriumphosphat), Na₂HPO₄, ist ein farbloses, sehr leicht wasserlösliches kristallines Salz. Es existiert wasserfrei und mit 2 Mol. (Dichte 2,066 gcm⁻³ Wasserverlust bei 95°), 7 Mol. (Dichte 1,68 gcm⁻³, Schmelzpunkt 48° unter Verlust von 5 H₂O) und 12 Mol. Wasser (Dichte 1,52 gcm⁻³, Schmelzpunkt 35° unter Verlust von 5 H₂O), wird bei 100° wasserfrei und geht bei stärkerem Erhitzen in das Diphosphat Na₄P₂O₇ über. Dinatriumhydrogenphosphat wird durch Neutralisation von Phosphorsäure mit Sodalösung unter Verwendung von Phenolphthalein als Indikator hergestellt. Dikaliumhydrogenphosphat (sekundäres od. zweibasiges Kaliumphosphat), K₂HPO₄, ist ein amorphes, weißes Salz, das in Wasser leicht löslich ist.

Trinatriumphosphat, tertiäres Natriumphosphat, Na₃PO₄, sind farblose Kristalle, die als Dodecahydrat eine Dichte von 1,62 gcm⁻³ und einen Schmelzpunkt von 73-76°C (Zersetzung), als Decahydrat (entsprechend 19-20% P₂O₅) einen Schmelzpunkt von 100°C und in wasserfreier Form (entsprechend 39-40% P₂O₅) eine Dichte von 2,536 gcm⁻³aufweisen. Trinatriumphosphat ist in Wasser unter alkalischer Reaktion leicht löslich und wird durch Eindampfen einer Lösung aus genau 1 Mol Dinatriumphosphat und 1 Mol NaOH hergestellt. Trikaliumphosphat (tertiäres oder dreibasiges Kaliumphosphat), K₃PO₄, ist ein weißes, zerfließliches, körniges Pulver der Dichte 2,56 gcm⁻³, hat einen Schmelzpunkt von 1340° und ist in Wasser mit alkalischer Reaktion leicht löslich. Es entsteht zum Beispiel beim Erhitzen von Thomasschlacke mit Kohle und Kaliumsulfat. Trotz des höheren Preises werden in der Reinigungsmittel-Industrie die leichter löslichen, daher hochwirksamen, Kaliumphosphate gegenüber entsprechenden Natrium-Verbindungen vielfach bevorzugt.

Tetranatriumdiphosphat (Natriumpyrophosphat), Na₄P₂O₇, existiert in wasserfreier Form (Dichte 2,534 gcm⁻³, Schmelzpunkt 988°, auch 880° angegeben) und als Decahydrat (Dichte 1,815-1,836 gcm⁻³, Schmelzpunkt 94° unter Wasserverlust). Beide Substanzen sind farblose, in Wasser mit alkalischer Reaktion lösliche Kristalle. Na₄P₂O₇ entsteht beim Erhitzen von Dinatriumphosphat auf >200°C oder indem man Phosphorsäure mit Soda im stöchiometrischem Verhältnis umsetzt und die Lösung durch Versprühen entwässert. Das Decahydrat komplexiert Schwermetall-Salze und Härtebildner und verringert daher die Härte des Wassers. Kaliumdiphosphat (Kaliumpyrophosphat), K₄P₂O₇, existiert in Form des Trihydrats und stellt ein farbloses, hygroskopisches Pulver mit der Dichte 2,33 gcm⁻³ dar, das in Wasser löslich ist, wobei der pH-Wert der 1%igen Lösung bei 25° 10,4 beträgt.

Durch Kondensation des NaH₂PO₄ beziehungsweise des KH₂PO₄ entstehen höhermolekulare Natrium- und Kaliumphosphate, bei denen man cyclische Vertreter, die Natrium- beziehungsweise Kaliummetaphosphate und kettenförmige Typen, die Natrium- beziehungsweise Kaliumpolyphosphate, unterscheiden kann. Insbesondere für letztere sind eine Vielzahl von Bezeichnungen in Gebrauch: Schmelz- oder Glühphosphate, Grahamsches Salz, Kurrolsches und Maddrellsches Salz. Alle höheren Natrium- und Kaliumphosphate werden gemeinsam als kondensierte Phosphate bezeichnet.

Das technisch wichtige Pentanatriumtriphosphat, Na₅P₃O₁₀ (Natriumtripolyphosphat), ist ein wasserfrei oder mit 6 H₂O kristallisierendes, nicht hygroskopisches, weißes, wasserlösliches Salz der allgemeinen Formel NaO-[P(O)(ONa)-O]ₙ-Na mit n=3. In 100 g Wasser lösen sich bei Zimmertemperatur etwa 17 g, bei 60° ca. 20 g, bei 100° rund 32 g des kristallwasserfreien Salzes; nach zweistündigem Erhitzen der Lösung auf 100° entstehen durch Hydrolyse etwa 8% Orthophosphat und 15% Diphosphat. Bei der Herstellung von Pentanatriumtriphosphat wird Phosphorsäure mit Sodalösung oder Natronlauge im stöchiometrischen Verhältnis zur Reaktion gebracht und die Lösung durch Versprühen entwässert. Ähnlich wie Grahamsches Salz und Natriumdiphosphat löst Pentanatriumtriphosphat viele unlösliche Metall-Verbindungen (auch Kalkseifen usw.). Pentakaliumtriphosphat, K₅P₃O₁₀ (Kaliumtripolyphosphat), kommt beispielsweise in Form einer 50 Gew.%-igen Lösung (> 23% P₂O₅, 25% K₂O) in den Handel. Die Kaliumpolyphosphate finden in der Wasch- und Reinigungsmittel-Industrie breite Verwendung. Weiter existieren auch Natriumkaliumtripolyphosphate, welche ebenfalls im Rahmen der vorliegenden Erfindung einsetzbar sind. Diese entstehen beispielsweise, wenn man Natriumtrimetaphosphat mit KOH hydrolysiert:

(NaPO₃)₃ + 2 KOH → Na₃K₂P₃O₁₀ + H₂O

Diese sind erfindungsgemäß genau wie Natriumtripolyphosphat, Kaliumtripolyphosphat oder Mischungen aus diesen beiden einsetzbar, auch Mischungen aus Natriumtripolyphosphat und Natriumkaliumtripolyphosphat oder Mischungen aus Kaliumtripolyphosphat und Natriumkaliumtripolyphosphat oder Gemische aus Natriumtripolyphosphat und Kaliumtripolyphosphat und Natriumkaliumtripolyphosphat sind erfindungsgemäß einsetzbar.

Als organische Cobuilder können in den erfindungsgemäßen Wasch- und Reinigungsmitteln insbesondere Polycarboxylate oder Polycarbonsäuren, polymere Polycarboxylate, Polyasparaginsäure, Polyacetale, gegebenenfalls oxidierte Dextrine, weitere organische Cobuilder (siehe unten) sowie Phosphonate eingesetzt werden. Diese Stoffklassen werden nachfolgend beschrieben.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu vermeiden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Auch die Säuren an sich können eingesetzt werden. Sie besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln, sofern nicht der sich durch die Mischung der übrigen Komponenten ergebende pH-Wert gewünscht ist. Insbesondere sind hierbei system- unbd umweltverträgliche Säuren wie Citronensäure, Essigsäure, Weinsäure, Äpfelsäure, Milchsäure, Glykolsäure, Bernsteinsäure, Glutarsäure, Adipinsäure, Gluconsäure und beliebige Mischungen aus diesen zu nennen. Aber auch Mineralsäuren, insbesondere Schwefelsäure oder Basen, insbesondere Ammonium- oder Alkalihydroxide können als pH-Regulatoren dienen. Derartige Regulatoren sind in den erfindungemäßen Mitteln in Mengen von vorzugsweise nicht über 20 Gew.-%, insbesondere von 1,2 Gew.-% bis 17 Gew.-%, enthalten.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70 000 g/mol.

Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2 000 bis 20 000 g/mol aufweisen. Aufgrund ihrer überlegenen Löslichkeit können aus dieser Gruppe wiederum die kurzkettigen Polyacrylate, die Molmassen von 2 000 bis 10 000 g/mol, und besonders bevorzugt von 3 000 bis 5 000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.% Maleinsäure enthalten. Ihre relative Molekülmasse, bezogen auf freie Säuren, beträgt im allgemeinen 2 000 bis 70 000 g/mol, vorzugsweise 20 000 bis 50 000 g/mol und insbesondere 30 000 bis 40 000 g/mol. Die (co-) polymeren Polycarboxylate können entweder als Pulver oder als wässerige Lösung eingesetzt werden. Der Gehalt der Mittel an (co-)polymeren Polycarboxylaten kann von 0,5 bis 20 Gew.-%, insbesondere 1 bis 10 Gew.-%, betragen.

Zur Verbesserung der Wasserlöslichkeit können die Polymere auch Allylsulfonsäuren, wie beispielsweise Allyloxybenzolsulfonsäure und Methallylsulfonsäure, als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol beziehungsweise Vinylalkohol-Derivate oder die als Monomere Salze der Acrylsäure und der 2-Alkylallylsulfonsäure sowie Zucker-Derivate enthalten.

Weitere bevorzugte Copolymere sind solche, die als Monomere vorzugsweise Acrolein und Acrylsäure/Acrylsäuresalze beziehungsweise Acrolein und Vinylacetat aufweisen.

Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren beziehungsweise deren Salze und Derivate.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere beziehungsweise Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2 000 bis 30 000 g/mol.

Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Besonders bevorzugte organische Builder für erfindungsgemäße Mittel sind oxidierte Stärken, beziehungsweise deren Derivate aus den Anmeldungen EP 472 042, WO 97/25399, und EP 755 944.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen zwischen 3 und 15 Gew.-%.

Weitere brauchbare organische Cobuilder sind beispielsweise acetylierte Hydroxycarbonsäuren beziehungsweise deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Cobuildereigenschaften stellen die Phosphonate dar. Dabei handelt es sich insbesondere um Hydroxyalkan- beziehungsweise Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, zum Beispiel als Hexanatriumsalz der EDTMP beziehungsweise als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüberhinaus können alle Verbindungen, die in der Lage sind, Komplexe mit Erdalkaliionen auszubilden, als Cobuilder eingesetzt werden.

Buildersubstanzen können in den erfindungsgemäßen Mitteln gegebenenfalls in Mengen bis zu 90 Gew.% enthalten sein. Sie sind vorzugsweise in Mengen bis zu 75 Gew.% enthalten. Erfindungsgemäße Waschmittel weisen Buildergehalte von insbesondere 5 Gew.-% bis 50 Gew.-% auf. In erfindungsgemäßen Mitteln für die Reinigung harter Oberflächen, insbesondere zur maschinellen Reinigung von Geschirr, beträgt der Gehalt an Buildersubstanzen insbesondere 5 Gew.% bis 88 Gew.-%, wobei in derartigen Mitteln vorzugsweise keine wasserunlöslichen Buildermaterialien eingesetzt werden. In einer bevorzugten Ausführungsform erfindungsgemäßer Mittel zur insbesondere maschinellen Reinigung von Geschirr sind 20 Gew.% bis 40 Gew.-% wasserlöslicher organischer Builder, insbesondere Alkalicitrat, 5 Gew.% bis 15 Gew.% Alkalicarbonat und 20 Gew.% bis 40 Gew.% Alkalidisilikat enthalten.

Lösungsmittel, die in den flüssigen bis gelförmigen Zusammensetzungen von Wasch- und Reinigungsmitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethyl-, -ethyl- oder -propyl-ether, Dipropylenglykolmonomethyl-, oder -ethylether, Di-isopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel.

Lösungsmittel können in den erfindungsgemäßen flüssigen bis gelförmigen Wasch- und Reinigungsmitteln in Mengen zwischen 0,1 und 20 Gew.%, bevorzugt aber unter 15 Gew.% und insbesondere unterhalb von 10 Gew.-% eingesetzt werden.

Zur Einstellung der Viskosität können erfindungsgemäßen Zusammensetzungen ein oder mehrere Verdicker, beziehungsweise Verdickungssysteme zugesetzt werden. Diese hochmolekularen Stoffe, die auch Quell(ungs)mittel genannt werden, saugen meist die Flüssigkeiten auf und quellen dabei auf, um schließlich in zähflüssige echte oder kolloide Lösungen überzugehen.

Geeignete Verdicker sind anorganische oder polymere organische Verbindungen. Zu den anorganischen Verdickem zählen beispielsweise Polykieselsäuren, Tonmineralien wie Montmorillonite, Zeolithe, Kieselsäuern und Bentonite. Die organischen Verdicker stammen aus den Gruppen der natürlichen Polymere, der abgewandelten natürlichen Polymere und der vollsynthetischen Polymere. Solche aus der Natur stammenden Polymere sind beispielsweise Agar-Agar, Carrageen, Tragant, Gummi arabicum, Alginate, Pektine; Polyosen, Guar-Mehl, Johannisbrotbaumkernmehl, Stärke, Dextrine, Gelatine und Casein. Abgewandelte Naturstoffe, die als Verdicker verwendet werden, stammen vor allem aus der Gruppe der modifizierten Stärken und Cellulosen. Beispielhaft seien hier Carboxymethylcellulose und andere Celluloseether, Hydroxyethyl- und -propylcellulose sowie Kernmehlether genannt. Vollsynthetische Verdicker sind Polymere wie Polyacryl- und Polymethacryl-Verbindungen, Vinylpolymere, Polycarbonsäuren, Polyether, Polyimine, Polyamide und Polyurethane.

Die Verdicker können in einer Menge bis zu 5 Gew.-%, vorzugsweise von 0,05 bis 2 Gew.%, und besonders bevorzugt von 0,1 bis 1,5 Gew.-%, bezogen auf die fertige Zusammensetzung, enthalten sein.

Das erfindungsgemäße Wasch- oder Reinigungsmittel kann gegebenenfalls als weitere übliche Inhaltsstoffe Sequestrierungsmittel, Elektrolyte und weitere Hilfsstoffe enthalten.

Erfindungsgemäße Textilwaschmittel können als optische Aufheller Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Um einen Silberkorrosionsschutz zu bewirken, können in erfindungsgemäßen Reinigungsmitteln für Geschirr Silberkorrosionsinhibitoren eingesetzt werden. Solche sind aus dem Stand der Technik bekannt, beispielsweise Benzotriazole, Eisen(III)-chlorid oder CoSO₄. Wie beispielsweise aus der europäischen Patentschrift EP 0 736 084 B1 bekannt ist, sind für die gemeinsame Verwendung mit Enzymen besonders geeignete Silberkorrosionsinhibitoren Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen. Beispiele für derartige Verbindungen sind MnSO₄, V₂O₅, V₂O₄, VO₂, TiOSO₄, K₂TiF₆, K₂ZrF₆, Co(NO₃)₂, Co(NO₃)₃, sowie deren Gemische.

"Soil-Release"-Wirkstoffe oder "Soil-Repellents" sind zumeist Polymere, die bei der Verwendung in einem Waschmittel der Wäschefaser schmutzabstoßende Eigenschaften verleihen und/oder das Schmutzablösevermögen der übrigen Waschmittelbestandteile unterstützen. Ein vergleichbarer Effekt kann auch bei deren Einsatz in Reinigungsmitteln für harte Oberflächen beobachtet werden.

Besonders wirksame und seit langer Zeit bekannte Soil-Release-Wirkstoffe sind Copolyester mit Dicarbonsäure-, Alkylenglykol- und Polyalkylenglykoleinheiten. Beispiele dafür sind Copolymere oder Mischpolymere aus Polyethylenterephthalat und Polyoxyethylenglykol (DT 16 17 141, beziehungsweise DT 22 00 911). In der deutschen Offenlegungsschrift DT 22 53 063 sind saure Mittel genannt, die unter anderem ein Copolymer aus einer dibasigen Carbonsäure und einem Alkylen- oder Cycloalkylenpolyglykol enthalten. Polymere aus Ethylenterephthalat und Polyethylenoxid-terephthalat und deren Einsatz in Waschmitteln sind in den deutschen Schriften DE 28 57 292 und DE 33 24 258 und der Europäischen Patentschrift EP 0 253 567 beschrieben. Das europäische Patent EP 066 944 betrifft Mittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyl- oder Ethylgruppen-endverschlossene Polyester mit Ethylen- und/oder Propylen-terephthalat- und Polyethylenoxid-terephthalat-Einheiten und Waschmitel, die derartiges Soil-release-Polymer enthalten, bekannt. Das europäische Patent EP 0 241 984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Aus der europäischen Patentanmeldung EP 0 272 033 sind zumindest anteilig durch C₁₋₄-Alkyl- oder Acylreste endgruppenverschlossene Polyester mit Polypropylenterephthalat- und Polyoxyethylenterephthalat-Einheiten bekannt. Das europäische Patent EP 0 274 907 beschreibt sulfoethyl-endgruppenverschlossene terephthalathaltige Soil-release-Polyester. Gemäß der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glykol-Einheiten hergestellt. Die internationale Patentanmeldung WO 95/32232 betrifft saure, aromatische schmutzablösevermögende Polyester. Aus der internationalen Patentanmeldung WO 97/31085 sind nicht polymere soil-repellent-Wirkstoffe für Materialien aus Baumwolle mit mehreren funktionellen Einheiten bekannt: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/ Baumwolle-Grenzfläche.

Zu den für den Einsatz in erfindungsgemäßen Textilwaschmitteln in Frage kommenden Farbübertragungsinhibitoren gehören insbesondere Polyvinylpyrrolidone, Polyvinylimidazole, polymere N-Oxide wie Poly-(vinylpyridin-N-oxid) und Copolymere von Vinylpyrrolidon mit Vinylimidazol.

Beim Einsatz in maschinellen Reinigungsverfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bistearylethylendiamid. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche, beziehungsweise dispergierbare Trägersubstanz gebunden. Insbesondere sind dabei Mischungen aus Paraffinen und Bistearylethylendiamiden bevorzugt.

Ein erfindungsgemäßes Reinigungsmittel für harte Oberflächen kann darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.-%, insbesondere von 5 Gew.-% bis 15 Gew.%, enthalten.

Farb- und Duftstoffe werden Wasch- und Reinigungsmitteln zugesetzt, um den ästhetischen Eindruck der Produkte zu verbessern und dem Verbraucher neben der Wasch- und Reinigungsleistung ein visuell und sensorisch "typisches und unverwechselbares" Produkt zur Verfügung zu stellen. Als Parfümöle beziehungsweise Duftstoffe können einzelne Riechstoffverbindungen, zum Beispiel die synthetischen Produkte vom Typ der Ester, Ether, Aldehyde, Ketone, Alkohole und Kohlenwasserstoffe verwendet werden. Riechstoffverbindungen vom Typ der Ester sind zum Beispiel Benzylacetat, Phenoxyethylisobutyrat, p-tert.-Butylcyclohexylacetat, Linalylacetat, Dimethylbenzyl-carbinylacetat, Phenylethylacetat, Linalylbenzoat, Benzylformiat, Ethylmethylphenyl-glycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Üblicherweise liegt der Gehalt von Wasch- und Reinigungsmitteln an Farbstoffen unter 0,01 Gew.%, während Duftstoffe bis zu 2 Gew.% der gesamten Formulierung ausmachen können.

Die Duftstoffe können direkt in die Wasch- und Reinigungsmittel eingearbeitet werden, es kann aber auch vorteilhaft sein, die Duftstoffe auf Träger aufzubringen, die die Haftung des Parfüms auf dem Reinigungsgut verstärken und durch eine langsamere Dufttreisetzung für langanhaltenden Duft, insbesondere von behandelten Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können. Ein weiter bevorzugter Träger für Duftstoffe ist der beschriebene Zeolith X, der anstelle von oder in Mischung mit Tensiden auch Duftstoffe aufnehmen kann. Bevorzugt sind daher Wasch- und Reinigungsmittel, die den beschriebenen Zeolith X und Duftstoffe, die vorzugsweise zumindest teilweise an dem Zeolithen absorbiert sind, enthalten.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Zur Bekämpfung von Mikroorganismen können Wasch- oder Reinigungsmittel antimikrobielle Wirkstoffe enthalten. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung, die beispielsweise von *K. H. Walthäußer* in "Praxis der Sterilisation, Desinfektion - Konservierung : Keimidentifizierung - Betriebshygiene" (5. Aufl. - Stuttgart; New York : Thieme, 1995) wiedergegeben wird, wobei alle dort beschriebenen Substanzen mit antimikrobieller Wirkung eingesetzt werden können. Geeignete antimikrobielle Wirkstoffe sind vorzugsweise ausgewählt aus den Gruppen der Alkohole, Amine, Aldehyde, antimikrobiellen Säuren beziehungsweise deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, lodo-2-propyl-butyl-carbamat, lod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, **i-Propanol,** 1,3-Butandiol, Phenoxyethanol, 1,2-Propylenglykol, Glycerin, Undecylensäure, Benzoesäure, Salicylsäure, Dihydracetsäure, o-Phenylphenol, N-Methylmorpholin-acetonitril (MMA), 2-Benzyl-4-chlorphenol, 2,2'-Methylen-bis-(6-brom-4-chlorphenol), 4,4'-Dichlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-hamstoff, N,N'-(1,10-decan-diyldi-1-pyridinyl-4-yliden)-bis-(1-octanamin)-dihydrochlorid, N,N'-Bis-(4-chlorphenyl)-3,12-diimino-2,4,11,13-tetraaza-tetradecandiimidamid, Glucoprotaminen, antimikrobiellen oberflächenaktiven quaternären Verbindungen, Guanidinen einschl. den Bi- und Polyguanidinen, wie beispielsweise 1,6-Bis-(2-ethylhexyl-biguanido-hexan)-dihydrochlorid, 1,6-Di-(N₁,N₁,'-phenyldiguanido-N₅, N₅')-hexan-tetrahydochlorid, 1,6-Di-(N₁, phenyl-N₁,N₁-methyldiguanido-N₅, N₅')-hexan-dihydrochlorid, 1,6-Di-(N₁,N₁'-o-chlorophenyldiguanido- N₅, N₅')-hexan-dihydrochlorid, 1,6-Di-(N₁,N₁'-2,6-dichlorophenyldiguanido-N₅,N_{5'})1,6-Di-[N₁,N₁'-beta-(p-methoxyphenyl) diguanido-N₅,N₅')-hexane-dihydrochlorid, 1,6-Di-(N₁,N₁'-alpha-methyl-beta-phenyldiguanido-N₅,N₅')-hexan-dihydrochlorid, 1,6-Di-(N₁,N₁'-p-nitrophenyldiguanido-N₅,N₅')hexan-dihydrochlorid,' omega:omega-Di-( N₁,N₁'-phenyldiguanido-N₅,N₅')-di-n-propylether-dihydrochlorid, omega:omega' -Di-(N₁, N₁' -p-chlorophenyldiguanido-N₅,N₅')-di-n-propylether-tetrahydrochlorid, 1,6-Di-(N₁,N₁'-2,4-dichlorophenyldiguanido-N₅, N₅')hexan-tetrahydrochlorid, 1,6-Di-(N₁,N₁,'-p-methylphenyldiguanido-N₅,N₅')hexan-dihydrochlorid, 1,6-Di-(N₁,N₁'-2,4,5-trichlorophenyldiguanido-N₅,N₅')hexan-tetrahydrochlorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophenyldiguanido-N₅,N₅)m-xylene-dihydrochlorid, 1,12-Di-(N₁,N₁'-p-chlorophenyldiguanido-N₅,N₅) dodecan-dihydrochlorid, 1,10-Di-(N₁,N₅'-phenyldiguanido- N₅,N₅')-decan-tetrahydrochlorid, 1,12-Di-(N₁,N₁'-phenyldiguanido- N₅,N₅') dodecan-tetrahydrochlorid, 1,6-Di-(N₁,N₁'-o-chlorophenyldiguanido- N₅,N₅') hexan-dihydrochlorid, 1,6-Di-(N₁,N₁'-o-chlorophenyldiguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbiguanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylen bis (o-tolylbiguanid), N-Butyltrimethyle- bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlor-meta-xylol, sowie natürliche antimikrobielle Wirkstoffe pflanzlicher Herkunft (zum Beispiel aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/oder mindestens ein natürlicher antimikrobieller Wirkstoff tierischer Herkunft aus der Gruppe, umfassend Enzyme wie Eiweiß aus Milch, Lysozym und Lactoperoxidase, und/oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden.

Die als antimikrobielle Wirkstoffe geeigneten quaternären Ammoniumverbindungen (QAV) weisen die allgemeine Formel (R¹)(R²)(R³)(R⁴) N⁺ X⁻ auf, in der R¹ bis R⁴ gleiche oder verschiedene C₁-C₂₂-Alkylreste, C₇-C₂₈-Aralkylreste oder heterozyklische Reste, wobei zwei oder im Falle einer aromatischen Einbindung wie im Pyridin sogar drei Reste gemeinsam mit dem Stickstoffatom den Heterozyklus, zum Beispiel eine Pyridinium- oder Imidazoliniumverbindung, bilden, darstellen und X- Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie zum Beispiel Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer MethylGruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielweise Benzalkoniumchlorid (N-Alkyl-N,N-dimethyl-benzyl-ammoniumchlorid, CAS No. 8001-54-5), Benzalkon B (m,p-Dichlorbenzyl-dimethyl-C12-alkyl-ammoniumchlorid, CAS No: 58390-78-6), Benzoxoniumchlorid (Benzyl-dodecyl-bis-(2-hydroxyethyl)-ammonium-chlorid), Cetrimoniumbromid (N-Hexadecyl-N, N-trimethyl-ammoniumbromid, CAS No. 57-09-0), Benzetoniumchlorid (N,N-Dimethyl-N-[2-[2-[p-(1,1,3,3-tetramethylbutyl)-pheno-xy]ethoxy]ethyl]-benzylammoniumchlorid, CAS No. 121-54-0), Dialkyldimethylammoniumchloride wie Di-n-decyl-dimethyl-ammoniumchlorid (CAS No. 7173-51-5-5), Didecyldimethylammoniumbromid (CAS No. 2390-68-3), Dioctyl-dimethyl-ammoniumchloric, 1-Cetylpyridiniumchlorid (CAS No. 123-03-5) und Thiazoliniodid (CAS No. 15764-48-1) sowie deren Mischungen. Besonders bevorzugte QAV sind die Benzalkoniumchloride mit C₈-C₁₈-Alkylresten, insbesondere C₁₂-C₁₄-Aklyl-benzyl-dimethyl-ammoniumchlorid.

Benzalkoniumhalogenide und/oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Die antimikrobiellen Wirkstoffe werden in Mengen von 0,0001 Gew.% bis 1 Gew.-%, bevorzugt von 0,001 Gew.% bis 0,8 Gew.%, besonders bevorzugt von 0,005 Gew.% bis 0,3 Gew.% und insbesondere von 0,01 bis 0,2 Gew.-% eingesetzt.

Die Mittel können UV-Absorbenzien (UV-Absorber) enthalten, die auf die behandelten Textilien aufziehen und die Lichtbeständigkeit der Fasern und/oder die Lichtbeständigkeit sonstiger Rezepturbestandteile verbessern. Unter UV-Absorber sind organische Substanzen (Lichtschutzflter) zu verstehen, die in der Lage sind, ultraviolette Strahlen zu absorbieren und die aufgenommene Energie in Form längerwelliger Strahlung, zum Beispiel Wärme wieder abzugeben.

Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate, gegebenenfalls mit Cyanogruppen in 2-Stellung), Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen: 3-Benzylidencampher beziehungsweise 3-Benzylidennorcampher und dessen Derivate, zum Beispiel 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-hydroxyl methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie zum Beispiel 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb^{®} HEB); Propan-1,3-dione, wie zum Beispiel 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie zum Beispiel 4-(2-Oxo-3-bornylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide beziehungsweise Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind ummantelte Titandioxide, wie zum Beispiel Titandioxid T 805 (Degussa) oder Eusolex® T2000 (Merck; als hydrophobe Coatingmittel kommen dafür bevorzugt Silicone und besonders bevorzugt Trialkoxyoctylsilane oder Simethicone in Frage. Vorzugsweise wird mikronisiertes Zinkoxid verwendet. Weitere geeignete UV-Lichtschutzfilter sind der Übersicht von P. Finkel in SÖFW Journal, Band 122 (1996), S. 543 zu entnehmen.

Die UV-Absorbenzien werden üblicherweise in Mengen von 0,01 Gew.% bis 5 Gew.%, vorzugsweise von 0,03 Gew.% bis 1 Gew.-%, eingesetzt.

Erfindungsgemäße Mittel können zur Steigerung der Wasch-, beziehungsweise Reinigungsleistung zusätzlich zu den erfindungsgemäß einsetzbaren Enzymen weitere Enzyme enthalten, wobei prinzipiell alle im Stand der Technik für diese Zwecke etablierten Enzyme einsetzbar sind. Hierzu gehören insbesondere Proteasen, Amylasen, Lipasen, Hemicellulasen, Cellulasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Erfindungsgemäße Mittel enthalten Enzyme vorzugsweise in Gesamtmengen von 1 x 10⁻⁶ bis 5 Gewichts-Prozent bezogen auf aktives Protein. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol Chem., 177 (1948), S. 751-766) bestimmt werden.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von der Firma Novozymes A/S, Bagsvaerd. Dänemark, erhältlich. Die Subtilisine 147 und 309 werden unter den Handelsnamen Esperase^{®}, beziehungsweise Savinase^{®} von der Firma Novozymes vertrieben. Von der Protease aus *Bacillus lentus* DSM 5483 (WO 91/02792 A1) leiten sich die unter der Bezeichnung BLAP^{®} geführten Varianten ab, die insbesondere in WO 92/21760 A1, WO 95/23221 A1, WO 02/088340 A2 und WO 03/038082 A2 beschrieben werden. Weitere verwendbare Proteasen aus verschiedenen *Bacillus sp.* und *B. gibsonii* gehen aus den Patentanmeldungen WO 03/054185 A1, WO 03/056017 A2, WO 03/055974 A2 und WO 03/054184 A1 hervor.

Weitere. brauchbare Proteasen sind beispielsweise die unter den Handelsnamen Durazym^{®}, Relase^{®}, Everlase^{®}, Nafizym, Natalase^{®}, Kannase^{®} und Ovozymes^{®} von der Firma Novozymes, die unter den Handelsnamen, Purafect^{®}, Purafect^{®} OxP und Properase^{®} von der Firma Genencor, das unter dem Handelsnamen Protosol^{®} von der Firma Advanced Biochemicals Ltd., Thane, Indien, das unter dem Handelsnamen Wuxi^{®} von der Firma Wuxi Snyder Bioproducts Ltd., China, die unter den Handelsnamen Proleather^{®} und Protease P^{®} von der Firma Amano Pharmaceuticals Ltd., Nagoya, Japan, und das unter der Bezeichnung Proteinase K-16 von der Firma Kao Corp., Tokyo, Japan, erhältlichen Enzyme.

Beispiele für erfindungsgemäß einsetzbare Amylasen sind die α-Amylasen aus *Bacillus licheniformis*, aus *B. amyloliquefaciens* oder aus *B. stearothermophilus* sowie deren für den Einsatz in Wasch- und Reinigungsmitteln verbesserte Weiterentwicklungen. Das Enzym aus *B. licheniformis* ist von der Firma Novozymes unter dem Namen Termamyl^{®} und von der Firma Genencor unter dem Namen Purastar^{®} ST erhältlich. Weiterentwicklungsprodukte dieser α-Amylase sind von der Firma Novozymes unter den Handelsnamen Duramyl^{®} und Termamyl^{®} ultra, von der Firma Genencor unter dem Namen Purastar^{®} OxAm und von der Firma Daiwa Seiko Inc., Tokyo, Japan, als Keistase^{®} erhältlich. Die α-Amylase von *B. amyloliquefaciens* wird von der Firma Novozymes unter dem Namen BAN^{®} vertrieben, und abgeleitete Varianten von der α-Amylase aus *B. stearothermophilus* unter den Namen BSG^{®} und Novamyl^{®}, ebenfalls von der Firma Novozymes.

Desweiteren sind für diesen Zweck die in der Anmeldung WO 02/10356 A2 offenbarte α-Amylase aus *Bacillus sp.* A 7-7 (DSM 12368) und die in der Anmeldung WO 02/44350 A2 beschriebene Cyclodextrin-Glucanotransferase (CGTase) aus *B. agaradherens* (DSM 9948) hervorzuheben. Ferner sind die amylolytischen Enzyme einsetzbar, die dem Sequenzraum von α-Amylasen angehören, der in der Anmeldung WO 03/002711 A2 definiert wird, und die, die in der Anmeldung WO 03/054177 A2 beschrieben werden. Ebenso sind Fusionsprodukte der genannten Moleküle einsetzbar, beispielsweise die aus der Anmeldung DE 10138753 A1.

Darüber hinaus sind die unter den Handelsnamen Fungamyl^{®} von der Firma Novozymes erhältlichen Weiterentwicklungen der α-Amylase aus *Aspergillus niger* und *A*. *oryzae* geeignet. Ein weiteres Handelsprodukt ist beispielsweise die Amylase-LT^{®}.

Erfindungsgemäße Mittel können, insbesondere wenn sie für die Behandlung von Textilien gedacht sind, Cellulasen enthalten, je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen. Zu diesen Leistungsaspekten zählen insbesondere Beiträge zur Primärwaschleistung, zur Sekundärwaschleistung des Mittels (Antiredepositionswirkung oder Vergrauungsinhibition) und Avivage (Gewebewirkung), bis hin zum Ausüben eines "stone washed"-Effekts.

Eine brauchbare pilzliche, Endoglucanase(EG)-reiche Cellulase-Präparation, beziehungsweise deren Weiterentwicklungen werden von der Firma Novozymes unter dem Handelsnamen Celluzyme^{®} angeboten. Die ebenfalls von der Firma Novozymes erhältlichen Produkte Endolase^{®} und Carezyme^{®} basieren auf der 50 kD-EG, beziehungsweise der 43 kD-EG aus *H. insolens* DSM 1800. Weitere einsetzbare Handelsprodukte dieser Firma sind Cellusoft^{®} und Renozyme^{®}. Letzteres basiert auf der Anmeldung WO 96/29397 A1. Leistungsverbesserte Cellulase-Varianten gehen beispielsweise aus der Anmeldung WO 98/12307 A1 hervor. Ebenso sind die in der Anmeldung WO 97/14804 A1 offenbarten Cellulasen einsetzbar; beispielsweise die darin offenbarte 20 kD-EG aus *Melanocarpus*, die von der Firma AB Enzymes, Finnland, unter den Handelsnamen Ecostone^{®} und Biotouch^{®} erhältlich ist. Weitere Handelprodukte der Firma AB Enzymes sind Econase^{®} und Ecopulp^{®}. Weitere geeignete Cellulasen aus *Bacillus sp.* CBS 670.93 und CBS 669.93 werden in WO 96/34092 A2 offenbart, wobei die aus *Bacillus sp.* CBS 670.93 von der Firma Genencor unter dem Handelsnamen Puradax^{®} erhältlich ist. Weitere Handelsprodukte der Firma Genencor sind "Genencor detergent cellulase L" und IndiAge^{®}Neutra.

Erfindungsgemäße Mittel können insbesondere zur Entfernung bestimmter Problemanschmutzungen weitere Enzyme enthalten, die unter dem Begriff Hemicellulasen zusammengefaßt werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullutanasen und β-Glucanasen. Geeignete Mannanasen sind beispielsweise unter den Namen Gamanase^{®} und Pektinex AR^{®} von der Firma Novozymes, unter dem Namen Rohapec^{®} B1L von der Firma AB Enzymes und unter dem Namen Pyrolase^{®} von der Firma Diversa Corp., San Diego, CA, USA erhältlich. Eine geeignete β-Glucanase aus einem *B. alcalophilus* geht beispielsweise aus der Anmeldung WO 99/06573 A1 hervor. Die aus *B. subtilis* gewonnene β-Glucanase ist unter dem Namen Cereflo^{®} von der Firma Novozymes erhältlich.

Zur Erhöhung der bleichenden Wirkung können erfindungsgemäße Wasch- und Reinigungsmittel Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) enthalten. Als geeignete Handelsprodukte sind Denilite^{®} 1 und 2 der Firma Novozymes zu nennen. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die in erfindungsgemäßen Mitteln eingesetzten Enzyme stammen entweder ursprünglich aus Mikroorganismen, etwa der Gattungen *Bacillus*, *Streptomyces*, *Humicola*, oder *Pseudomonas*, und/oder werden nach an sich bekannten biotechnologischen Verfahren durch geeignete Mikroorganismen produziert, etwa durch transgene Expressionswirte der Gattungen *Escherichia coli, Bacillus*, *Streptomyces* oder filamentöse Fungi.

Die Aufreinigung der betreffenden Enzyme erfolgt günstigerweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte.

Erfindungsgemäßen Mitteln können die Enzyme in jeder nach dem Stand der Technik etablierten Form zugesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so daß ein einzelnes Granulat mehrere Enzymaktivitäten aufweist.

Die auf die enthaltenen Enzyme, insbesondere auf die enthaltenen Polyol-Oxidasen, zurückzuführende Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem. 177 (1948), S. 751-766) bestimmt werden.

Ein in einem erfindungsgemäßen Mittel enthaltenes Protein und/oder Enzym kann besonders während der Lagerung gegen Schädigungen wie beispielsweise Inaktivierung, Denaturierung oder Zerfall etwa durch physikalische Einflüsse, Oxidation oder proteolytische Spaltung geschützt werden. Bei mikrobieller Gewinnung der Proteine und/oder Enzyme ist eine Inhibierung der Proteolyse besonders bevorzugt, insbesondere wenn auch die Mittel Proteasen enthalten. Bevorzugte erfindungsgemäße Mittel enthalten zu diesem Zweck Stabilisatoren.

Eine Gruppe von Stabilisatoren sind reversible Proteaseinhibitoren. Häufig werden hierfür Benzamidin-Hydrochlorid, Borax, Borsäuren, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin. Auch spezifische, reversible Peptid-Inhibitoren für die Protease Subtilisin sowie Fusionsproteine aus Proteasen und spezifischen Peptid-Inhibitoren sind hierfür geeignet.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Bestimmte als Builder eingesetzte organische Säuren vermögen, wie in WO 97/18287 offenbart, zusätzlich ein enthaltenes Enzym zu stabilisieren.

Niedere aliphatische Alkohole, vor allem aber Polyole, wie beispielsweise Glycerin, Ethylenglykol, Propylenglykol oder Sorbit sind weitere häufig eingesetzte Enzymstabilisatoren. Auch Di-Glycerinphosphat schützt gegen Denaturierung durch physikalische Einflüsse. Ebenso werden Calcium- und/oder Magnesiumsalze eingesetzt, wie beispielsweise Calciumacetat oder Calcium-Formiat.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind lineare C₈-C₁₈ Polyoxyalkylene. Auch Alkylpolyglycoside können die enzymatischen Komponenten des erfindungsgemäßen Mittels stabilisieren und vermögen vorzugsweise, diese zusätzlich in ihrer Leistung zu steigern. Vernetzte N-haltige Verbindungen erfüllen vorzugsweise eine Doppelfunktion als Soil-release-Agentien und als Enzym-Stabilisatoren. Hydrophobes, nichtionisches Polymer stabilisiert insbesondere eine gegebenenfalls enthaltene Cellulase.

Reduktionsmittel und Antioxidantien erhöhen die Stabilität der Enzyme gegenüber oxidativem Zerfall; hierfür sind beispielsweise schwefelhaltige Reduktionsmittel geläufig. Andere Beispiele sind Natrium-Sulfit und reduzierende Zucker, aber auch Katalase in geeignet geringer Konzentration.

Besonders bevorzugt werden Kombinatonen von Stabilisatoren eingesetzt, beispielsweise aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen. Die Wirkung von Peptid-Aldehyd-Stabilisatoren wird günstigerweise durch die Kombination mit Borsäure und/oder Borsäurederivaten und Polyolen gesteigert und noch weiter durch die zusätzliche Wirkung von zweiwertigen Kationen, wie zum Beispiel Calcium-Ionen.

Erfindungsgemäße Mittel sind in einer bevorzugten Ausführungsform dadurch gekennzeichnet, daß sie, beispielsweise um die enthaltenen Wirkstoffe zeitlich oder räumlich voneinander getrennt freizusetzen, aus mehr als einer Phase bestehen. Dabei kann es sich um Phasen in verschiedenen, insbesondere aber um Phasen in denselben Aggregatzuständen handeln.

Die erfindungsgemäßen Mittel liegen vorzugsweise als ein- oder mehrphasige, insbesondere zweiphasige flüssige und versprühbare Mittel vor, welche insbesondere in einer Flasche mit Sprühsystem angewendet werden. Beim Versprühen der Mittel wird besonders gut mit Sauerstoff gesättigt, weswegen man von einem besonderem Vorteil für eine Enzym-Bleiche bzw. Desinfektion sprechen kann.

Die mehrphasigen flüssigen und versprühbaren Mittel (Sprays) werden entweder über eine Mehrkammerflasche mit Sprühsystem angewendet oder sie werden vor dem Gebrauch mehrfach geschüttelt, um eine temporäre Emulsion herzustellen.

Organische Lösungsmittel, welche geeignet sind, die Zweiphasigkeit der Mittel zu bewirken, sind beispielsweise Kohlenwasserstoffe und Alkylether. Als Kohlenwasserstoffe sind insbesondere solche bevorzugt, welche einen Siedepunkt von oberhalb 150 °C und vorzugsweise oberhalb von 180 °C aufweisen. Als Alkylether kommen insbesondere Dialkylether, vor allem C₆-C₁₈-Alkylether mit besonderer Bevorzugung der C₈-C₁₂-Alkylether, beispielsweise Dioctylether, in Betracht.

Zu weiteren organischen Lösungsmitteln, die auch eine hervorragende Reinigungswirkung aufweisen, gehören Butoxypropoxypropanole (BPP), welche als Mischung mehrerer Isomeren kommerziell erhältlich sind. Da BPP nicht vollständig mit Wasser mischbar sind, sind sie insbesondere dazu geeignet, in mehrphasigen Mitteln eingesetzt zu werden. Sollen BPP jedoch in einphasigen Mitteln auf wässeriger Basis eingesetzt werden, so ist es erforderlich, zusätzlich Emulgatoren einzusetzen. Für Beispiele möglicher Emulgatoren wird auf die Offenbarung der WO 96/30580 verwiesen.

Die erfindungsgemäßen Mittel können Tenside und zwar anionische, nichtionische, kationische, zwitterionische, amphotere Tenside oder beliebige Mischungen aus diesen enthalten. Der Gehalt der Mittel an Tensiden kann in einem breiten Bereich variieren. Auch kleinere Mengen, die aber immer noch eine schmutzdispergierende und damit reinigungsverstärkende Wirkung zeigen, können enthalten sein; der Gehalt der Mittel an Tensiden liegt beispielsweise in einem Bereich von 0,2 bis 10 Gew.-%, wobei Gehalte von 0,5 bis 10 Gew.-% und insbesondere von 1 bis 8 Gew.-% an anionischen und/oder nichtionischen Tensiden besonders bevorzugt ist. Auch amphotere Tenside und/oder kationische Tenside sind für die Verwendung in der vorliegenden Erfindung bevorzugt und können zusätzliche als Textilweichmacher wirken.

Zusätzlich können die Mittel Duftstoffe, Desodorantien, Konservierungsstoffe, antibakterielle Wirkstoffe, Insektenabschreckungsmittel (Mottenschutzmittel) und/oder Färbemittel enthalten wie auch ein oder mehrere Appreturmittel, Gleitmittel, Fungizide etc., solange diese Additive nicht die Verteilung des Mittels stören. Auch ph-regulierende Substanzen wie beispielsweise organische Säuren, insbesondere Citronensäure, Bernsteinsäure, Glutarsäure, Adipinsäure, Gluconsäure und beliebige Mischungen aus diesen, können enthalten sein. Die Mengen dieser Additive umfassen - wenn vorhanden - im allgemeinen etwa 0,2 bis 5 Gew.-% des gesamten Mittels.

Die flüssigen und versprühbaren Mittel werden vorzugsweise in einer Flasche mit einem Sprühsystem eingesetzt. Flaschen mit Sprühsystem sind dem Fachmann aus dem Stand der Technik bekannt. Insbesondere geeignet sind Flaschen mit einem Pump- oder Trigger-Spraybehältnis, welche es erlauben, einen feinen und konzentrierten Sprühstrahl zielgerichtet auf das Textil zu applizieren. Die Flaschen können aus jedem für derartige Zwecke bekannten Material hergestellt werden; üblicherweise handelt es sich um Kunststoff- und Plastikflaschen, wobei Polyethylen-, und Polypropylen-Werkstoffe bevorzugt sind. Der Fachmann kennt aber auch andere Werkstoffe, die ebenso geeignet sind. Zum Schutz der Sprühvorrichtung kann die Flasche eine Steckkappe aufweisen. Auch hier sind dem Fachmann Ausführungsformen aus dem Stand der Technik bekannt. Auch sind Pumpen geeignet, die bei ihrer Anwendung Schäume erzeugen. Eine derartige Schaumerzeugungsvorrichtung ist als "F2 Finger Pump Foamer" der Firma Airspray^{®} im Markt bekannt. Weiters eignet sich in besonderer Weise ein manuell aktivierter Sprühspender, insbesondere ausgewählt aus der Gruppe, umfassend Aerosolsprühspender, selbst Druck aufbauende Sprühspender, Pumpsprühspender und Triggersprühspender, insbesondere Pumpsprühspender und Triggersprühspender mit einem Behälter aus Polyethylen oder Polyethylenterephthalat. Solche Triggerflaschen werden beispielsweise von der Firma Afa-Polytec angeboten. Der Sprühkopf ist vorzugsweise mit einer Schaumdüse ausgestattet. Weiterhin sind dem Fachmann aus dem Stand der Technik Einhebel- oder Einarm-Spender bekannt, die einen Flüssigkeitsbehälter und eine Schaumerzeugungsvorrichtung umfassen und umhüllen. Eine verbesserte Ausführungsform eines derartigen Schaumspenders wird in der deutschen Patentanmeldung 199 51 011.3 beschrieben.

Erfindungsgemäße Mittel, die aus mehr als einer festen Komponente zusammengesetzt sind, können auf einfache Weise dadurch hergestellt werden, daß verschiedene feste Komponenten, insbesondere Pulver, Granulate oder Extrudate mit verschiedenen Inhaltsstoffen und/oder unterschiedlichem Freisetzungsverhalten in insgesamt loser Form miteinander vermischt werden. Die Herstellung erfindungsgemäßer fester Mittel aus einer oder mehreren Phasen kann auf bekannte Weise, zum Beispiel durch Sprühtrocknen oder Granulation erfolgen, wobei die Enzyme und eventuelle weitere thermisch empfindliche Inhaltsstoffe wie zum Beispiel Bleichmittel gegebenenfalls später separat zugesetzt werden. Zur Herstellung erfindungsgemäßer Mittel mit erhöhtem Schüttgewicht, insbesondere im Bereich von 650 g/l bis 950 g/l, ist ein aus der europäischen Patentschrift EP 0 486 592 bekanntes, einen Extrusionschritt aufweisendes Verfahren bevorzugt. Eine weitere bevorzugte Herstellung mit Hilfe eines Granulationsverfahrens ist in der europäischen Patentschrift EP 0 642 576 beschrieben.

Proteine können für feste Mittel beispielsweise in getrockneter, granulierter, verkapselter oder verkapselter und zusätzlich getrockneter Form eingesetzt werden. Sie können separat, das heißt als eigene Phase, oder mit anderen Bestandteilen zusammen in derselben Phase, mit oder ohne Kompaktierung zugesetzt werden. Sollen mikroverkapselte Enzyme in fester Form verarbeitet werden, so kann das Wasser mit aus dem Stand der Technik bekannten Verfahren aus den sich aus der Aufarbeitung ergebenden wäßrigen Lösungen entfernt werden, wie Sprühtrocknung, Abzentrifugieren oder durch Umsolubilisieren. Die auf diese Weise erhaltenen Teilchen haben üblicherweise eine Teilchengröße zwischen 50 und 200 µm.

Die verkapselte Form bietet sich an, um wie oben bereits diskutiert, auch die Enzyme vor anderen Bestandteilen, wie beispielsweise Bleichmitteln, zu schützen oder um eine kontrollierte Freisetzung (controlled release) zu ermöglichen. Je nach der Größe dieser Kapseln wird nach Milli-, Mikro- und Nanokapseln unterschieden, wobei Mikrokapseln für Enzyme besonders bevorzugt sind. Solche Kapseln werden beispielsweise mit den Patentanmeldungen WO 97/24177 und DE 199 18 267 offenbart. Eine weitere mögliche Verkapselungsmethode besteht darin, daß die für die Verwendung in Wasch- oder Reinigungsmitteln geeigneten Enzyme, ausgehend von einer Mischung der Enzymlösung mit einer Lösung oder Suspension von Stärke oder einem Stärkederivat, in Stärke, beziehungsweise dem Stärkederivat verkapselt werden. Ein solches Verkapselungsverfahren wird mit der deutschen Anmeldung DE 199 56 382 beschrieben.

Es können auch mindestens zwei feste Phasen miteinander verbunden vorliegen. So besteht eine Möglichkeit, ein festes erfindungsgemäßes Mittel zur Verfügung zu stellen, in dem Verpressen oder Kompaktieren zu Tabletten. Solche Tabletten können ein- oder mehrphasig sein. Damit bietet auch diese Darreichungsform die Möglichkeit, ein festes erfindungsgemäßes Mittel mit zwei festen Phasen vorzulegen. Zur Herstellung von erfindungsgemäßen Mitteln in Tablettenform, die einphasig oder mehrphasig, einfarbig oder mehrfarbig sein können und/oder aus einer mehreren Schichten bestehen können, werden vorzugsweise alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN/cm², vorzugsweise bei 60 bis 70 kN/cm² verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN/cm², insbesondere bei 10 bis 15 kNcm² durchgeführt. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind.

Besonders vorteilhaft ist es, wenn in mehrphasigen Mitteln wenigstens eine der Phasen ein Amylase-sensitives Material, insbesondere Stärke enthält oder von diesem zumindest teilweise umgeben oder beschichtet ist. Auf diese Weise wird diese Phase mechanisch stabilisiert und/oder gegen Einflüsse von außen geschützt und gleichzeitig über eine in der Waschflotte wirksame Amylase angegriffen, so daß die Freisetzung der Inhaltsstoffe erleichtert wird.

Ebenfalls bevorzugte erfindungsgemäße Mittel sind dadurch gekennzeichnet, daß sie insgesamt flüssig, gelförmig oder pastös vorliegen. Die enthaltenen Proteine, vorzugsweise ein erfindungsgemäßes Protein, werden solchen Mitteln bevorzugt ausgehend von einer nach dem Stand der Technik durchgeführten Proteingewinnung und Präparation in konzentrierter wäßriger oder nichtwäßriger Lösung, beispielsweise in flüssiger Form, etwa als Lösung, Suspension oder Emulsion, aber auch in Gelform oder verkapselt oder als getrocknetes Pulver zugesetzt. Derartige erfindungsgemäße Wasch- oder Reinigungsmittel in Form von Lösungen in üblichen Lösungsmitteln werden in der Regel durch einfaches Mischen der Inhaltsstoffe hergestellt, die in Substanz oder als Lösung in einen automatischen Mischer gegeben werden können.

Eine Ausführungsform der vorliegenden Erfindung sind solche flüssigen, gelförmigen oder pastösen Mittel, denen ein erfindungswesentliches Protein und/oder eines der anderen enthaltenen Proteine und/oder einer der anderen enthaltenene Inhaltsstoffe verkapselt, vorzugsweise in Form von Mikrokapseln zugesetzt worden ist. Besonders bevorzugt sind darunter solche mit Kapseln aus amylasesensitivem Material. Solch eine gemeinsame Verwendung von Amylase-sensitiven Materialien und einem amylolytischen Enzym in einem Wasch- oder Reinigungsmittel kann Synergieeffekte zeigen, etwa dergestalt daß das stärkespaltende Enzym die Spaltung der Mikrokapseln unterstützt und somit den Freisetzungsprozeß der verkapselten Inhaltsstoffe steuert, so daß deren Freisetzung nicht während der Lagerung und/oder nicht zu Beginn des Reinigungsvorgangs, sondern erst zu einem bestimmten Zeitpunkt erfolgt. Auf diesem Mechanismus können komplexe Wasch- und Reinigungsmittelsysteme mit verschiedensten Inhaltsstoffen und verschiedensten Kapseltypen beruhen, die besonders bevorzugte Ausführungsformen der vorliegenden Erfindung darstellen.

Ein vergleichbarer Effekt ist dann gegeben, wenn sich die Inhaltsstoffe des Wasch- oder Reinigungsmittels auf mindestens zwei unterschiedliche Phasen verteilen, beispielsweise zwei oder mehr feste, miteinander verbundene Phasen eines tablettenförmigen Wasch- oder Reinigungsmittels, oder verschiedene Granulate innerhalb desselben pulverförmigen Mittels. Zwei- oder Mehrphasenreiniger sind für die Anwendung sowohl in maschinellen Geschirrspülern als auch in Waschmitteln Stand der Technik. Die Aktivität eines amylolytischen Enzyms in einer früher aktivierten Phase ist Voraussetzung für die Aktivierung einer späteren Phase, wenn diese von einer Amylase-sensitiven Hülle oder Beschichtung umgeben ist oder das Amylase-sensitive Material einen integralen Bestandteil der festen Phase darstellt, bei dessen teilweiser oder vollständiger Hydrolyse die betreffende Phase desintegriert.

Die Inhaltsstoffe von Wasch- und Reinigungsmitteln vermögen sich geeigneterweise gegenseitig in ihrer Leistung zu unterstützen. So ist auch aus der Anmeldung WO 98/45396 bekannt, daß Polymere, die gleichzeitig als Cobuilder eingesetzt werden können, wie beispielsweise Alkyl-Poly-Glykoside, die Aktivität und die Stabilität von enthaltenen Enzymen stabilisieren und steigern können. Somit ist es bevorzugt, wenn eine erfindungsgemäße Polyoloxidase durch einen der übrigen, oben aufgeführten Bestandteile modifiziert, insbesondere stabilisiert und/oder in seinem Beitrag zur Wasch-, beziehungsweise Reinigungsleistung des Mittels gesteigert wird.

Einen weiteren Erfindungsgegenstand stellen Verfahren zur Reinigung von Textilien oder von harten Oberflächen dar, die dadurch gekennzeichnet sind, daß in wenigstens einem der Verfahrensschritte eine oben beschriebene erfindungsgemäße Polyol-Oxidase aktiv wird.

Denn in dieser Ausführungsform wird die Erfindung dadurch realisiert, daß die erfindungsgemäß verbesserten enzymatischen Eigenschaften hinsichtlich einer Verbesserung prinzipiell jeden Reinigungsverfahrens ausgenutzt werden. Jedes Reinigungsverfahren wird um die betreffende Aktivität bereichert, wenn sie in wenigstens einem Verfahrensschritt zugesetzt wird. Derartige Verfahren werden beispielsweise mit Maschinen wie gängigen Haushaltsgeschirrspülmaschinen oder Haushaltswaschmaschinen verwirklicht. Bevorzugte Verfahren werden entsprechend den oben gemachten Angaben bevorzugt. Weiter bevorzugt sind derartige Verfahren, die dadurch gekennzeichnet sind, daß die Polyol-Oxidase über ein oben beschriebenes Mittel eingesetzt wird.

Ein weiterer Gegenstand der Erfindung ist ein Haarwasch- und/oder Haarpflegemittel, enthaltend erfindungsgemäß einsetzbare Polyol-Oxidasen.

Die Haarwasch- und/oder Haarpflegemittel sowie Schaumbäder, Duschbäder, Cremes, Gele, Lotionen, alkoholische und wäßrig/alkoholische Lösungen, Emulsionen, Wachs/ Fett-Massen, Stiftpräparate, Puder oder Salben, die erfindungsgemäß verwendbare Polyol-Oxidasen umfassen, können als Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Licht-schutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, □-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Olkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkyl- und/oder Alkenylmono- und -oligoglycoside mit 8 bis 22 Kohlenstoffatomen im Alk(en)ylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid).sowie Polyglucoside (z. B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 PS als Rückfettungsmittel für kosmetische Zubereitungen bekannt.

Alkyl- und/oder Alkenylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N, N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Januar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylaceta/ButylmaleaT/ Isobornylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproyl-methacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

Typische Beispiele für Fette sind Glyceride, als Wachse kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als keimhemmende Mittel, die gegebenenfalls den erfindungsgemäßen Kosmetika zugesetzt werden können, sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Menthol, Minzöl, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Enzyminhibitoren können den erfindungsgemäßen Kosmetika ebenfalls zugesetzt werden. Beispielsweise sind Esteraseinhibitoren möglicherweise geeignete Enzyminhibitoren. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw - phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
(a) adstringierende Wirkstoffe,
(b) Ölkomponenten,
(c) nichtionische Emulgatoren,
(d) Coemulgatoren,
(e) Konsistenzgeber,
(f) Hilfsstoffe wie z. B. Verdicker oder Komplexierungsmittel und/oder
(g) nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als Antischuppenmittel können Climbazol, Octopirox und Zinkpyrethion eingesetzt werden.

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der EP 0693471 B1 beschrieben;
- 4-Aminobenzoesäurederivate, vorzugsweise 4-Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
- Propan-1,3-dione, wie z.B. 1-(4-tert.Butylphenyl)-3-4'methoxyphenyl)propan-1,3-dion;
- Ketotricyclo(5.2.1.0)decan-Derivate, wie in der EP 0694521 B1 beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Ein weiterer Gegenstand der Erfindung ist ein Oxidationsfärbemittel zum Färben von Keratinfasern, enthaltend erfindungsgemäß verwendbare Polyol-Oxidasen und insbesondere erfindungsgemäße Polyol-Oxidasen. Als Keratinfasern sind dabei Wolle, Federn, Pelze und insbesondere menschliche Haare zu verstehen.

Zur Herstellung der erfindungsgemäßen Oxidationsmittel werden die Oxidationsfarbstoffvorprodukte sowie die Polyol-Oxidasen in einen geeigneten wäßrigen Träger unter Ausschluss von Luftsauerstoff eingearbeitet. Solche Träger sind z. B. verdickte wäßrige Lösungen, Cremes (Emulsionen), Gele oder tensidhaltige schäumende Zubereitungen, z. B. Shampoos oder Schaumaerosole oder andere Zubereitungen, die für die Anwendung auf dem Haar geeignet sind.

Grundsätzlich sind auch wasserfreie Pulver als Träger geeignet; in diesem Falle werden die Oxidationsfärbemittel unmittelbar vor der Anwendung in Wasser gelöst oder dispergiert. Als Trägerkomponenten werden bevorzugt
- Netz- und Emulgiermittel
- Verdickungsmittel
   Reduktionsmittel (Antioxidantien)
- haarpflegende Zusätze
- Duftstoffe und
- Lösungsmittel wie z. B. Wasser, Glycole oder niedere Alkohole
eingesetzt.

Als Netz- und Emulgiermittel eignen sich z. B. anionische, zwitterionische, ampholytische und nichtionische Tenside. Auch kationische Tenside können zur Erzielung bestimmter Effekte eingesetzt werden.

Als Verdickungsmittel eignen sich die wasserlöslichen hochmolekularen Polysaccharid-Derivate oder Polypep-tide, z. B. Cellulose- oder Stärkeether, Gelatine, Pflanzengumme, Biopolymere (Xanthan-Gum) oder wasserlösliche synthetische Polymere wie z. B. Polyvinylpyrrolidon, Polyvinylalkohol, Polyethylenoxide, Polyacrylamide, Polyurethane, Polyacrylate und andere.

Weiterhin kann man tensidhaltige Zubereitungen auch durch Solubilisierung oder Emulgierung von polaren Lipiden verdicken. Solche Lipide sind z. B. Fettalkohole mit 12-18 C-Atomen, (freie) Fettsäuren mit 12-18 C-Atomen, Fettsäurepartialglyceride, Sorbitanfettsäureester, Fettsäurealkanolamide, niedrig oxethylierte Fettsäuren oder Fettalkohole, Lecithine, Sterine. Schließlich kann man gelförmige Träger auch auf Basis wässriger Seifengele, z. B. von Ammonium-Oleat, erzeugen.

Reduktionsmittel (Antioxidantien), die dem Träger zugesetzt werden, um eine vorzeitige oxidative Entwicklung des Farbstoffs vor der Anwendung auf dem Haar zu verhindern, sind z. B. Natriumsulfit oder Natriumascorbat.

Haarpflegende Zusätze können z.B. Fette, Öle oder Wachse in emulgierter Form, strukturgebende Additive wie z. B. Glucose oder Pyridoxin, avivierende Komponenten wie z. B. wasserlösliche Proteine, Proteinabbauprodukte, Aminosäuren, wasserlösliche kationische Polymere, Silicone, Vitamine, Panthenol oder Pflanzenextrakte sein.

Schließlich können Duftstoffe und Lösungsmittel wie z. B. Glycole wie 1,2-Propylenglycole, Glycerin, Glycolether wie z. B. Butylglycol, Ethyldiglycol oder niedere einwertige Alkohole wie Ethanol oder Isopropanol enthalten sein.

Zusätzlich können noch weitere Hilfsmittel enthalten sein, die die Stabilität und Anwendungseigenschaften der Oxdationsfärbemittel verbessern, z. B. Komplexbildner wie EDTA, NTA oder Organophosphonate, Quell- und Penetrationsmittel wie z. B. Harnstoff, Guanidin, Hydrogencarbonate, Puffersalze wie z. B. Ammoniumchlorid, Ammoniumcitrat, Ammoniumsulfat oder Alkanolammoniumsalze und gegebenenfalls Treibgase.

Ein weiterer Gegenstand der Erfindung ist ein Mittel zur Mund-, Zahn- oder Zahnprothesenpflege, insbesondere Prothesenreiniger, enthaltend erfindungsgemäße Polyol-Oxidasen zur Bleiche oder zur Desinfektion.

Bei Teilprothesen bzw. Gebissen eignet sich sowohl die Darreichung als Gebissreinigungstabletten, als auch als Mundspülung bzw. Mundwasser oder als Zahnpasta.

Die erfindungsgemäßen Mund-, Zahn- und/oder Zahnprothesenpflegemittel können beispielsweise als Mundwasser, Gel, flüssige Zahnputzlotion, steife Zahnpaste, Gebissreiniger oder Prothesenhaftcreme vorliegen.

Hierzu ist es erforderlich, die erfindungsgemäß einsetzbaren Polyol-Oxidasen in einen geeigneten Träger einzuarbeiten.

Als Träger können z.B. auch pulverförmige Zubereitungen oder wässrig-alkoholische Lösungen dienen, die als Mundwässer 0 bis 15 Gew.-% Ethanol, 1 bis 1,5 Gew.-% Aromaöle und 0,01 bis 0,5 Gew.-% Süßstoffe oder als Mundwasser-Konzentrate 15 bis 60 Gew.-% Ethanol, 0,05 bis 5 Gew.-% Aromaöle, 0,1 bis 3 Gew.-% Süßstoffe sowie ggf. weitere Hilfsstoffe enthalten können und vor Gebrauch mit Wasser verdünnt werden. Die Konzentration der Komponenten muss dabei so hoch gewählt werden, dass nach Verdünnung die genannten Konzentrationsuntergrenzen bei der Anwendung nicht unterschritten werden.

Als Träger können aber auch Gele sowie mehr oder weniger fließfähige Pasten dienen, die aus flexiblen Kunststoffbehältern oder Tuben ausgedrückt und mit Hilfe einer Zahnbürste auf die Zähne aufgetragen werden. Solche Produkte enthalten höhere Mengen an Feuchthaltemitteln und Bindemitteln oder Konsistenzreglern und Polierkomponenten. Darüber hinaus sind auch in diesen Zubereitungen Aromaöle, Süßstoffe und Wasser enthalten.

Als Feuchthaltemittel können dabei z.B. Glycerin, Sorbit, Xylit, Propylenglykole, Polyethenylenglycole oder Gemische dieser Polyole, insbesondere solche Polyethenylenglycole mit Molekulargewichten von 200 bis 800 (von 400 - 2000) verwendet werden enthalten sein. Bevorzugt ist als Feuchthaltemittel Sorbit in einer Menge von 25 - 40 Gew.-% enthalten.

Als Antizahnstein-Wirkstoffe und als Demineralisierungs-Inhibitoren können kondensierten Phosphate in Form ihrer Alkalisalze, bevorzugt in Form ihrer Natrium- oder Kaliumsalze enthalten sein. Die wäßrigen Lösungen dieser Phosphate reagieren aufgrund hydrolytischer Effekte alkalisch. Durch Säurezusatz wird der pH-Wert der erfindungsgemäßen Mund-, Zahn- und/oder Zahnprothesenpflegemittel auf die bevorzugten Werte von 7,5 - 9 eingestellt.

Es können auch Gemische verschiedener kondensierter Phosphate oder auch hydratisierte Salze der kondensierten Phosphate eingesetzt werden. Die spezifizierten Mengen von 2 - 12 Gew.-% beziehen sich jedoch auf die wasserfreien Salze. Bevorzugt ist als kondensiertes Phosphat ein Natrium- oder Kalium-tripolyphosphat in einer Menge von 5 - 10 Gew.-% der Zusammensetzung enthalten.

Ein bevorzugt enthaltener Wirkstoff ist eine karieshemmende Fluorverbindung, bevorzugt aus der Gruppe der Fluoride oder Monofluorophosphate in einer Menge von 0,1 - 0,5 Gew.-% Fluor. Geeignete Fluorverbindungen sind z.B. Natriummonofluorophosphat (Na₂PO₃F), Kaliummonofluorophosphat, Natrium- oder Kaliumfluorid, Zinnfluorid oder das Fluorid einer organischen Aminoverbindung.

Als Bindemittel und Konsistenzregler dienen z.B. natürliche und synthetische wasserlösliche Polymere wie Carragheen, Traganth, Guar, Stärke und deren nichtionogene Derivate wie z.B. Hydroxypropylguar, Hydroxyethylstärke, Celluloseether wie z.B. Hydroxyethylcellulose oder Methylhydroxypropylcellulose. Auch Agar-Agar, Xanthan-Gum, Pektine, wasserlösliche Carboxyvinylpolymere (z.B. Carbopol^{®}-Typen), Polyvinylalkohol, Polyvinylpyrrolidon, höhermolekulare Polyethylenglykole (Molekulargewicht 10³ bis 10 D). Weitere Stoffe, die sich zur Viskositätskontrolle eignen, sind Schichtsilikare wie z.B. Montmorillonit-Tone, kolloidale Verdickungskieselsäuren, z.B. Aerogel-Kieselsäure oder pyrogene Kieselsäuren.

Als Polierkomponenten können alle hierfür bekannten Poliermittel, bevorzugt aber Fällungs- und Gelkieselsäuren, Aluminiumhydroxid, Aluminiumsilicat, Aluminiumoxid, Aluminiumoxidtrihydrat, unlösliches Natriummetaphosphat, Calciumpyrophosphat, Calciumhydrogenphosphat, Dicalciumphosphat, Kreide, Hydroxylapatit, Hydrotalcite, Talkum, Magnesiumaluminiumsilicat (Veegum^{®}), Calciumsulfat, Magnesiumcarbonat, Magnesiumoxid, Natriumaluminiumsilikate, z.B. Zeolith A oder organische Polymere, z.B. Polymethacrylat, eingesetzt werden. Die Poliermittel werden vorzugsweise in kleineren Mengen von z.B. 1 - 10 Gew.-% verwendet.

Die erfindungsgemäßen Zahn- und/oder Mundpflegeprodukte können durch Zugabe von Aromaölen und Süßungsmitteln in ihren organoleptischen Eigenschaften verbessert werden. Als Aromaöle kommen alle für Mund-, Zahn- und/oder Zahnprothesenpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Kümmelöl, Eukalyptusöl, Fenchelöl, Zimtöl, Geraniumöl, Salbeiöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton. Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

Als Tenside sind dabei insbesondere Alkyl- und/oder Alkenyl-(oligo)-glycoside einsetzbar. Ihre Herstellung und Verwendung als oberflächenaktive Stoffe sind beispielsweise aus US-A-3 839 318, US-A-3 707 535, US-A-3 547 828 DE-A- 19 43 689, DE-A-20 36 472 und DE-A-30 01 064 sowie EP-A-77 167 bekannt. Bezüglich des Glycosidrestes gilt, daß sowohl Monoglycoside (x = 1), bei denen ein Pentose- oder Hexoserest glycosidisch an einen primären Alkohol mit 4 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl- und/oder Alkenyl-(oligo)-glycosid ein Alkyl- und/oder Alkenyl-(oligo)-glucosid der Formel RO(C₆H₁₀O)ₓ-H, in der R eine Alkyl- und/oder Alkenyl-gruppe mit 8 bis 14 C-Atomen ist und x einen Mittelwert von 1 bis 4 hat. Besonders bevorzugt sind Alkyloligoglucoside auf Basis von gehärtetem C_{12/14}-Kokosalkohol mit einem DP von 1 bis 3. Das Alkyl- und/oder Alkenyl-glycosid-Tensid kann sehr sparsam verwendet werden, wobei bereits Mengen von 0,005 bis 1 Gew.-% ausreichend sind.

Außer den genannten Alkylglucosid-Tensiden können auch andere nichtionische, ampholytische und kationische Tenside enthalten sein, als da beispielsweise sind: Fettalkoholpolyglycolethersulfate, Monoglyceridsulfate, Monoglyceridethersulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, Alkylamido-betaine und/oder Proteinfettsäurekondensate, letztere vorzugsweise auf Basis von Weizenproteinen. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mund-, Zahn- und/oder Zahnprothesenpflegemittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Rizinusöl (d.h. an Oxystearinsäure- oder Ricinolsäure-triglycerid), an Glyzerin-mono- und/oder -distearat oder an Sorbitanmono- und/oder - distearat.

Weitere übliche Zusätze für die Mund-, Zahn- und/oder Zahnprothesenpflege mittel sind z.B.
- Pigmente, z.B. Titandioxid, und/oder Farbstoffe
- pH-Stellmittel und Puffersubstanzen wie z.B. Natriumbicarbonat, Natriumcitrat, Natriumbenzoat, Zitronensäure, Phosphorsäure oder saure Salze, z.B. NaH₂PO₄
- wundheilende und entzündungshemmende Stoffe wie z.B. Allantoin, Harnstoff, Panthenol, Azulen bzw. Kamillenextrakt
- weitere gegen Zahnstein wirksame Stoffe wie z.B. Organophosphonate, z.B. Hydroxyethandiphosphonate oder Azacycloheptandiphosphonat
- Konservierungsstoffe wie z.B. Sorbinsäure-Salze, p-Hydroxybenzoesäure-Ester.
- Plaque-Inhibitoren wie z.B. Hexachlorophen, Chlorhexidin, Hexetidin, Triclosan, Bromchlorophen, Phenylsalicylsäureester.

In einer besonderen Ausführungsform ist die Zusammensetzung eine Mundspülung, ein Mundwasser, ein Prothesenreiniger oder ein Prothesenhaftmittel.

Für erfindungsgemäß bevorzugte Prothesenreiniger, insbesondere Prothesenreinigungstabletten und -pulver, eignen sich neben den schon genannten Inhaltsstoffen für die Mund-, Zahn- und/oder Zahnprothesenpflege zusätzlich noch Per-Verbindungen wie beispielsweise Peroxoborat, Peroxomonosulfat oder Percarbonat. Sie haben den Vorteil, dass sie neben der Bleichwirkung gleichzeitig auch desodorierend und/oder desinfizierend wirken. Der Einsatz solcher Per-Verbindungen in Prothesenreinigern beträgt zwischen 0,01 und 10 Gew.-%, insbesondere zwischen 0,5 und 5 Gew.-%.

Als weitere Inhaltsstoffe sind auch Enzyme, wie z.B. Proteasen und Carbohydrase, zum Abbau von Proteinen und Kohlenhydraten geeignet. Der pH-Wert kann zwischen pH 4 und pH 12, insbesondere zwischen pH 5 und pH 11 liegen.

Für die Prothesenreinigungstabletten sind zusätzlich noch weitere Hilfsstoffe notwendig, wie beispielsweise Mittel, die einen sprudelnden Effekt hervorrufen, wie z.B. CO₂ freisetzende Stoffe wie Natriumhydrogencarbonat, Füllstoffe, z.B. Natriumsulfat oder Dextrose, Gleitmittel, z.B. Magnesiumstearat, Fließregulierungsmittel, wie beispielsweise kolloidales Siliziumdioxid und Granuliermittel, wie die bereits erwähnten hochmolekularen Polyethylenglykole oder Polyvinylpyrrolidon.

Prothesenhaftmittel können als Pulver, Cremes, Folien oder Flüssigkeiten angeboten werden und unterstützen die Haftung der Prothesen.

Als Wirkstoffe sind natürliche und synthetische Quellstoffe geeignet. Als natürliche Quellstoffe sind neben Alginaten auch Pflanzengummen, wie z.B. Gummi arabicum, Traganth und Karaya-Gummi sowie natürlicher Kautschuk aufzufassen. Insbesondere haben sich Alginate und synthetische Quellstoffe, wie z.B. Natriumcarboxymethylcellulose, hochmolekulare Ethylenoxid-Copolymere, Salze der Poly(vinyl-ether-co-maleinsäure) und Polyacrylamide.

Als Hilfsstoffe für pastöse und flüssige Produkte eignen sich besonders hydrophobe Grundlagen, insbesondere Kohlenwasserstoffe, wie beispielsweise Weißes Vaselin (DAB) oder Paraffinöl.

Die folgenden Beispiele erläutern die Erfindung, ohne sie jedoch darauf einzuschränken:
Homologien des Wildtyp-Gens und -Proteins aus Seq. 3 mit anderen bekannten Sequenzen aus Datenbanken

Die Sequenzen, die dem im Beispiel untersuchten Enzym zugrundeliegen, wurden mit BLASTN bzw. BLASTP in der NR-Datenbank bei www.ncbi.nlm.nih.gov zur Suche nach homologen Sequenzabschnitten verwendet. Die Sequenzen mit den höchsten Scores wurden dann als komplette Proteinsequenz bzw. komplette codierende DNA-Sequenz gespeichert und jeweils mit den Sequenzen von Seq. 4 im paarweisen Alignment unter www.ebi.ac.uk/emboss/align/ hinsichtlich des Homologiegrades untersucht (Parameter: Matrix: Blosum62, Gap Extend: 0.5, Gap Open: 10). Die nächsten Homologen Sequenzeinträge waren:

### Homologien auf Proteinsequenz-Ebene

| **Datenbank-Eintrag** | **% Identität** | **% Homologie** |
|---|---|---|
| gi\|29605736\|dbj\|BAC69801.1 putative xylitol oxidase [Streptomyces avermitilis MA-4680] | 79,8 | 84,7 |
| gi\|7678808\|dbj\|BAA95146.1 xylitol oxidase [Streptomyces sp.\| | 75,7 | 82,3 |
| gi\|7481907\|pir\| JW0076 sorbitol oxidase (EC 1.-.-.-) Streptomyces sp. | 57,5 | 66,2 |
| gi\|48837117\|ref ZP_00294112.1\| COG0277: FAD/FMN-containing dehydrogenases [Thermobifida fusca] | 32,2 | 44,8 |

### Homologien auf DNA-Sequenz-Ebene

| **Datenbank-Eintrag** | **% Identität** |
|---|---|
| gi\|57546753:2547752-2549020 Streptomyces avermitilis MA-4680 qenomic DNA, complete genome | 80,7 |
| gi\|7678807\|dbj\|D89822.1\| Streptomyces sp. xyoA gene for xylitol oxidase, complete cds | 36,3 |
| gi\|1856966\|dbj\|AB000519.1\| Streptomyces sp. DNA for sorbitol oxidase, complete cds | 56,3 |

Hierunter befinden sich erwartungsgemäß auch die literaturbekannten Proteine zu Seq. 1 und Seq. 2.

### Beispiel 1:

### Klonierung, heterologe Überexpression, Aufreinigung und Charakterisierung einer ZuckeralkoholOxidase aus Streptomyces coelicolor A3(2) DSMZ Nr. 40783

Soweit nicht anders vermerkt werden die Teilschritte nach den in Sambrook, J., Fritsch, E.F. and Maniatis, T. 2001. *Molecular cloning: a laboratory manual*, 3. Edition Cold Spring Laboratory Press, beschriebenen Standardmethoden durchgeführt.

### Klonierung

Chromosomale DNA von *S*. *coelicolor A3(2)* DSMZ-Nr. 40783 wurde präpariert. Der für die putative Xylitol-Oxidase codierende DNA-Abschnitt (Genbank-Eintrag D89822, Seq.-Anhang 3) wurde mit Hilfe der Primer 5'-TTAGGATCCATGAGCGACATCACGGTCACCAACTGG-3' und 5'-ATTAAGCTTCCGCGAGCACCCCGCGCACGAACG-3' durch Polymerase-Kettenreaktion amplifiziert, wobei durch die Primer *Bam*H1 und *Hind*III-Schnittstellen angefügt wurden. Das Insert wurde in pQE80L (Qiagen, Hilden) ligiert, der durch diese Klonierungsstrategie einen N-terminalen Hexahistidin-Tag N-terminal zum codierten Protein anfügt. Nach Transformation des Plasmids in den Expressionsstamm *E*. *coli* BL21(DE3)Gold und Selektion eines positiven Klons wurde das Plasmid isoliert und der gesamte inserierte Teil sequenziert.

### Expression und Reinigung

Die Expression des heterologen Gens erfolgte in 100 mL LB-Medium mit Hilfe des Overnight Express Autoinduction Kits (Novagen), Inkubation bei 30°C und 215 rpm in einem 500 mL Erlenmeyerkolben mit Schikanen. Nach 24 Stunden wurde die Zellmasse durch Zentrifugation abgetrennt. Das Zellpellet wurde in 20 mL BugBuster-Reagenz aufgelöst und anschließend durch Zentrifugation geklärt. Die Aufreinigung erfolgte durch Nickel-IMAC (immobilised metal affinity chromatography) unter Nutung des N-terminalen Tags in Anlehnung an die Anweisungen des Herstellers (Qiagen, Hilden) für die nicht-denaturierende Aufreinigung. Die spezifische Aktivität des Eluats lag über 5 U/mg, die Reinheit wurde aus dem SDS-PAGE nach Coomassie-Färbung auf 95% geschätzt.

### Assay

Der Assay beruht auf einer photometrischen Bestimmung der gebildeten Wasserstoffperoxidmenge mit Hilfe eines Systems aus 4-Aminoantipyrin, Chromotropsäure und Meerrettich-Peroxidase.

150 µL Enzymprobe wird mit 250 µL Assay-Mix (160 mM Substrat, 0,16 mM Hydroxylaminsulfat als Katalaseinhibitor in 50 mM Glycin-NaOH Puffer pH 10) für 10 Minuten unter Schütteln bei 37°C inkubiert. Dann gibt man 500 µL Peroxidreagenz (12,5 mM Chromotropsäure, 0.6 mM 4-Aminoantipyrin in 200 mM Phosphatpuffer pH 5) zu und startet die Farbreaktion durch Zugabe von 75 µL Peroxidaselösung (52 U/mL in 200 mM Phosphatpuffer pH 5). Die Absorption wird bei 600 nm gemessen.

### Biochemische Charakterisierung

### 1. Allgemeine Charakterisierung

Das Enzym hat eine apparente Molmasse von 48 kDa auf dem SDS-PAGE; die berechnete Molmasse mit dem HisTag und kovalent gebundenem FAD beträgt 45,855 kDa. Das UV/VIS-Spektrum ist das eines typischen FAD-haltigen Enzyms, und das Verhältnis der Absorptionen bei 280 nm und 450 nm zeigt ein Mol FAD pro Mol Enzym an (ε_{450-FAD} angenommen als 11,3 mM⁻¹cm⁻¹, ε_{280-FAD} angenommen als: 20,6 mM⁻¹cm⁻¹, ε_{280-Protein} berechnet: 50,31 mM⁻¹cm⁻¹).

### 2. Substratspezifität

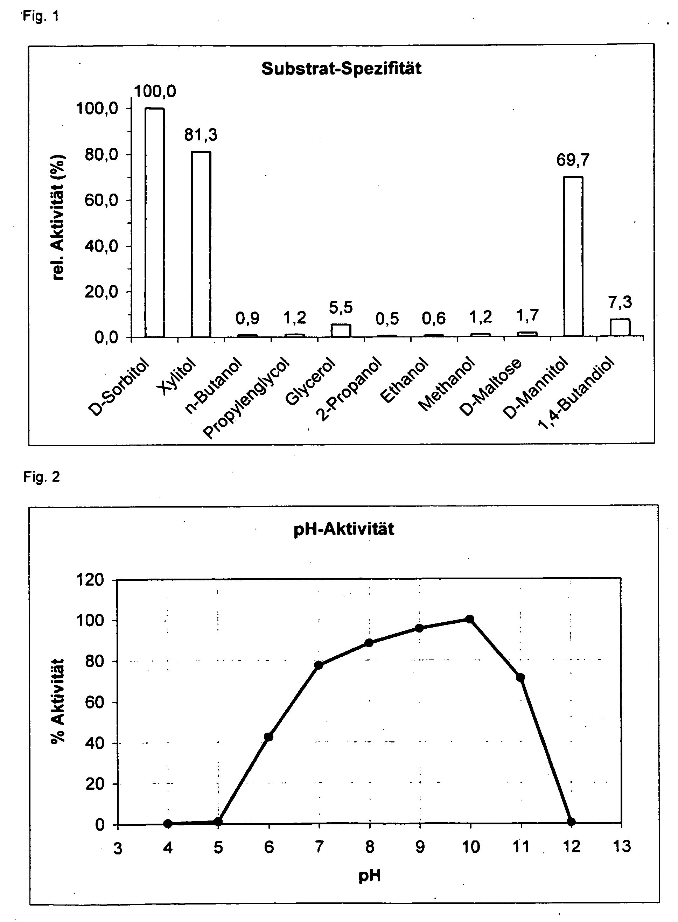

Die Substratspezifität wurde bestimmt, indem in dem o.a. Assay Sorbitol durch das jeweils zu testende Substrat äquimolar ersetzt wurde. Das Enzym akzeptiert die getesteten, in Fig. 1 aufgezählten Substrate mit der dort angegebenen relativen Aktivität bezogen auf das beste Substrat D-Sorbitol. Überraschenderweise wurde also gefunden, daß das vorliegende Enzym keine Xylitol- sondern eine Sorbitoloxidase ist, entgegen den Vorhersagen aus Sequenzhomologien.

### 3. pH-Aktivitätskurve

Die pH-Abhängigkeit der Enzymaktivität wurde mit dem o.a. Assay bestimmt, indem als Puffer Davies-Puffer auf den gewünschten pH eingestellt wurde und die Aktivität bestimmt wurde. Das Kurvenmaximum wurde auf 100% gesetzt. Die Ergebnisse sind in Fig. 2 dargestellt.

Die pH-Aktivitätskurve unterscheidet sich deutlich von der der beschriebenen Sorbitoloxidase (Oda et al., Hiraga *et al*.), die bei dem für Bleiche ungeeigneten pH 6,5 bis 7,5 ihr Optimum hat. Die von Yamashita *et al.* beschriebene Xylitol-Oxidase hat ebenfalls ihr Aktivitätsmaxium bei pH 7,5.

### 4. pH-Stabilitätskurve

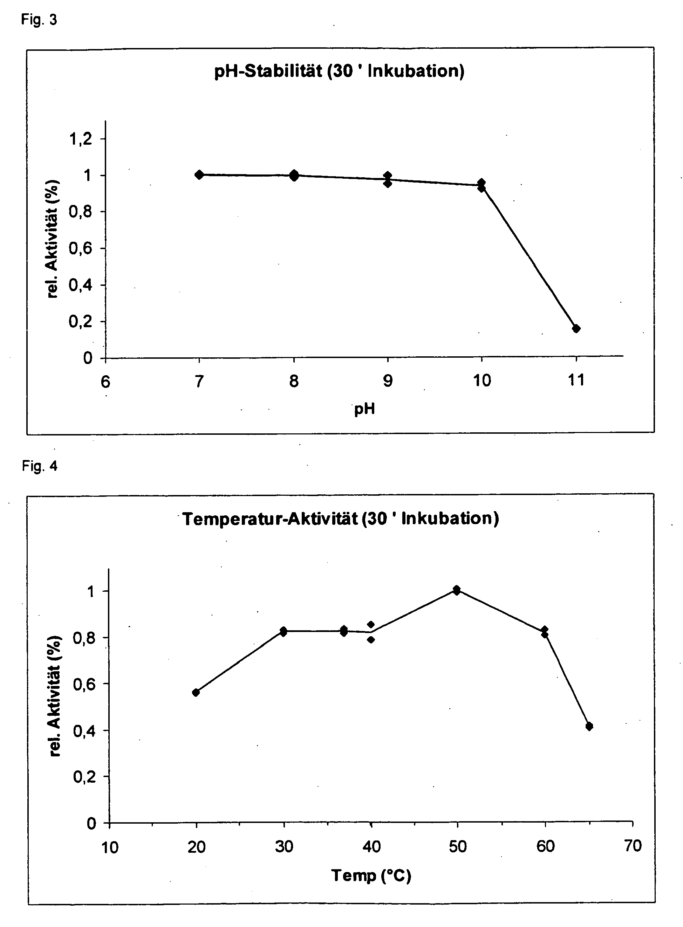

Zur Messung der Stabilität des Enzyms bei unterschiedlichen pH-Werten wurde das Enzym in einer Konzentration von 0,26 U/mL bei 37°C 30 min. in Davies-Puffer des entsprechenden pH-Wertes ohne Substrat vorinkubiert, bevor mit dem Assay die Aktivität bestimmt wurde. Die Ergebnisse sind in Fig. 3 dargestellt.

### 5. Temperatur-Aktivitätskurve

Zur Bestimmung der Temperaturabhängigkeit der Enzymaktivität wurde der Assay bei unterschiedlichen Temperaturen durchgeführt. Die Ergebnisse sind in Fig. 4 dargestellt.

### 6. Temperatur-Stabilitätskurve

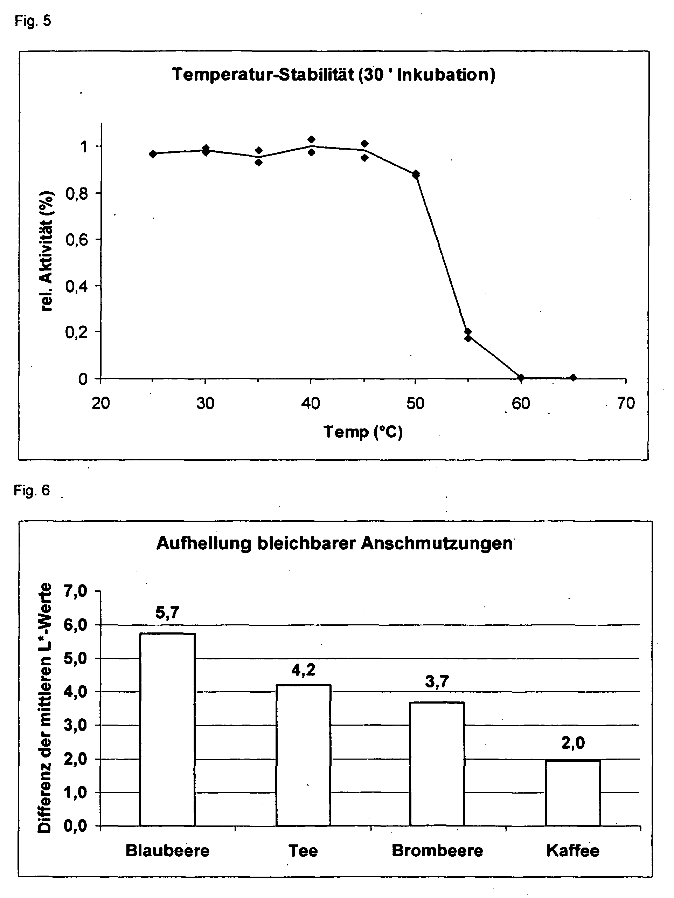

Die Stabilität bei unterschiedlichen Temperaturen wurde im Aktivitätsassay gemessen, nachdem das Enzym in einer Konz. von 2,6 U/mL bei unterschiedlichen Temperaturen für 30 min. in Glycin-Puffer, pH 10, in Abwesenheit von Substrat vorinkubiert wurde. Die Ergebnisse sind in Fig. 5 dargestellt.

### 7. Beladung mit dem Cofaktor FAD

Die Beladung mit dem Cofaktor FAD ist bereits im o.g. Genbank-Eintrag aufgrund der Homologien zu FAD-haltigen Enzymen vorhergesagt. Die von der Anmelderin gemessenen Absorptionsmaxima entsprechen denen in der zit. Lit. für FAD genannten und unterstützen diese Vorhersage. Es ist im Genbank-Eintrag auch die kovalente Bindung an einen Histidin-Rest aus Sequenzhomologien abgeleitet worden. Durch Denaturierung des Enzyms in 8-molarer Harnstofflösung, Ausfällung des Proteins mit Trichloressigsäure, Zentrifugation und Wiederaufnahme des Pellets in 8-molarer Harnstofflösung konnte nachgewiesen werden, daß bei der Denaturierung des Proteins kein FAD frei in Lösung gegangen ist, sondern das FAD vollständig in der Proteinfraktion verblieb. Die UV/VIS-Spektren des Proteins in Harnstofflösung vor und nach der Behandlung waren gleich. Dadurch wird eine kovalente Bindung des Cofaktors an das Enzym belegt.

### Beispiel 2:

### Erzeugung von Wasserstoffperoxid in Waschmittellösung

Zur Darstellung der erfindungsgemäßen Einsetzbarkeit des Enzyms in Waschmittel wurde Persil Kraftgel (Rezeptur Compact TL Gel 0255) 1:242 verdünnt, so daß die in der Waschlauge herrschende Konzentration vorlag, und diese Waschlauge wurde im Assay statt des dort angegebenen Reaktionspuffers verwendet. Das Enzym zeigte hierin eine ca. zweifach höhere Aktivität als im Standard-Assay, insofern ist das Enzym hervorrangend für Flüssigwaschmittel einsetzbar.

### H₂O₂-Erzeugung in Zahnpflegemittel mit Hilfe einer Zuckeralkoholoxidase

Die Tatsache, daß durch Wasserstoffperoxid Zähne aufgehellt werden können, ist dem Fachmann bekannt, und im folgenden Beispiel soll anhand der Sorbitoloxidase gezeigt werden, wie in einer Zahncreme-Formulierung im Munde während des Putzens Wasserstoffperoxid generiert werden kann. Dieses Wasserstoffperoxid kann desinfizierende oder zahnaufhellende Wirkung haben.

### Durchführung

1 g Zahncreme (Theramed Original, Fa. Henkel) wurden mit 2 mL 100 mM Kaliumphosphatpuffer, pH 8,0 und 1 mL 640 mM Sorbitollösung in vorgenanntem Puffer wurden mit einem Magnetrührer bei Raumtemperatur gerührt, mit Sorbitoloxidase versetzt und in Minutenabstand die Wasserstoffperoxid-Konzentration bestimmt. Die 1:4 Verdünnung, die der Zahncreme widerfuhr, simulierte die Verdünnung der Zahncreme mit Speichel während des Putzvorganges. Die Wasserstoffperoxid-Bestimmung erfolgte nach 1:10-Verdünnung mit bidest. Wasser mit Peroxid-Meßstreifen der Firma Merck KGaA im Merck Reflectoquant RQflex2 des Meßbereichs 0,2-20mg/L.

Ergebnisse:

| Rührzeit (min.) | mg/L Wasserstoffperoxid in der Zahncreme-Verdünnung |
|---|---|
| 0 | <1 |
| 5 | 2 |
| 10 | 5 |
| 15 | 6 |

Es wurden also (ermittelt durch lineare Regression durch den Ursprung) 430 µg/L Wasserstoffperoxid pro Minute in der Testlösung generiert.

### Aufhellung von bleichbaren Anschmutzungen

Bleichbare Anschmutzungen auf Baumwollgewebe (z.B. Tee, Kaffee, Brombeere, Blaubeere; z.B. von CFT, EMPA, WFK oder Henkel) wurden mit 1 cm Durchmesser ausgestanzt, mit 3 mL Waschlauge überschichtet und 30 min. bei Temperaturen zwischen 30°C und 60°C 50 mal pro Minute 180° um die Horizontale geschwenkt. Anschließend wurden die Gewebe unter fließendem Leitungswasser abgespült und über Nacht an der Luft getrocknet. Es wurde dann die Lichtreflexion gemessen und die Bleichleistung wie folgt ermittelt: Der Weißheitsgrad der gewaschenen Textilien im Vergleich zu einem Weißstandard (d/8, 8mm, SCI/SCE), der auf 100% normiert worden war, wurde gemessen. Die Messung erfolgte an einem Farbmessgerät (Minolta Cm508d) mit einer Lichtarteinstellung von 10°/D65. Die erhaltenen Ergebnisse werden als Prozent Remission, das heißt als Prozentangabe im Vergleich zum Weißstandard zusammen mit den jeweiligen Anfangswerten zusammengestellt. Die Bleichleistung ergibt sich als L*-Wert der Differenz der mittleren Remissionswerte von Basiswaschmittel mit bzw. ohne Zuckeralkoholoxidase unter gleichen Versuchsbedingungen.

Die Waschversuche erfolgten in Doppelbestimmungen, und der jeweilige Mittelwert wurde verwendet.

Als Waschmittel, das mit Zuckeralkoholoxidasen zur Aufhellung verwendet werden kann, kommen z.B. Mittel folgender Zusammensetzung in Betracht:

| Substanz | Rahmenrezeptur (% Reinsubstanz) |
|---|---|
| Verdicker | 0,5-2 |
| Antischaummittel | 0,1-0,4 |
| Glycerin / Propylenglykol | 5-10 |
| Ethanol | 0,1-2 |
| FAEOS | 2-7 |
| Nonionisches Tensid (FAEO, APG, u.a.) | 5-10 |
| Borsäure | 0,5-2 |
| Sodium citrat *2H₂O | 1-4 |
| Caustic soda | 2-6 |
| Kokosnußfettsäure | 0-10 |
| Phosphonate | 0,2-2 |
| Farbtransferinhibitor | 0-1 |
| optische Aufheller | 0-0,5 |
| Farbe | 0-0,001 |
| Parfüm | 0-2 |
| H₂O, demin. | Rest |
| Anionische Tenside | 0-20 |

Der pH wurde z.B. mit Natriumcarbonat auf 10 eingestellt und Bleichaktivator zwischen 0 und 10 % hinzugegeben.

In einem konkreten Beispiel wurden Blaubeer-, Tee-, Brombeer- und Kaffeeanschmutzungen bei 37°C mit Waschlauge entsprechend obiger Zusammensetzung gewaschen, die mit Natriumcarbonat auf pH 10 eingestellt und mit einem enzymatischen Bleichsystem, bestehend aus 100 mM Sorbitol, 10 U/mL Sorbitoloxidase und 4,5% TAED, versetzt war. Jeweils zum Vergleich wurde dasselbe Waschmittel bei pH 10 ohne das enzymatische Bleichsystem getestet. Die Bleichleistung in Form der Differenz der relativen Helligkeitswerte zwischen Waschmittel und Waschmittel mit enzymatischem Bleichsystem sind in Fig. 6 dargestellt.

Die im Beispiel beschriebene Sorbitol-Oxidase weist einen ausgesprochen breiten pH-Bereich mit Aktivität auf, mit nutzbarer Aktivität im pH-Bereich 5,5 bis 11,5; 80% Aktivität im pH-Bereich 7-10,5. Das Aktivitätsoptimum liegt bei pH 10. Diese pH-Kurve deckt z.B. ideal den Bereich von Maschinen- und Handspülmitteln sowie Waschmitteln ab, woraus auch ein Vorteil der universellen Einsetzbarkeit erwächst. Die Substratspezifität für Sorbitol mit starker Nebenaktivität für Xylitol ermöglicht den kostengünstigeren Einsatz von Sorbitol bei weiterhin erhaltener Möglichkeit, statt dessen auch Xylitol oder eine Mischung zu verwenden, wenn die Umsetzung von Xylitol bevorzugt wird, z.B. im Bereich der Mundhygiene. Die biochemischen Daten des Enzyms (s. Beispiel) belegen ferner, daß das im Beispiel behandelte Enzym sich in besonderer Weise im Sinne der Erfindung eignet, da die biochemischen Daten besser zu den Anforderungen v.a. in Wasch- und Reinigungsmitteln mit alkalischem pH passen als dies bei den Literaturbekannten Sorbitol- und Xylitoloxidasen der Fall ist.

### Abbildungen

In Fig. 1 sind die Werte der relativen Aktivität von Sorbitol im Vergleich zu anderen getesteten Substraten angegeben. Sorbitot wurde durch das jeweils zu testende Substrat äquimolar ersetzt. Das Enzym akzeptiert die getesteten, in Fig. 1 aufgezählten Substrate mit der dort angegebenen relativen Aktivität bezogen auf das beste Substrat D-Sorbitol. Überraschenderweise wurde also gefunden, daß das vorliegende Enzym keine Xylitol- sondern eine Sorbitoloxidase ist.

In Fig. 2 ist die pH-Abhängigkeit der Enzymaktivität dargestellt. Als Puffer wurde Davies-Puffer verwendet und der gewünscht pH-Wert eingestellt, als Substrat wurde Sorbitol verwendet. Das Kurvenmaximum wurde auf 100% gesetzt.

In Fig. 3 ist die pH-Stabilität des Enzyms dargestellt. Das Enzym wurde in einer Konzentration von 0,26 U/mL bei 37°C 30 min. in Davies-Puffer des entsprechenden pH-Wertes ohne Substrat vorinkubiert, bevor mit dem Assay die Aktivität bestimmt wurde.

In Fig. 4 sind die Ergebnisse zur Temperaturabhängigkeit der Aktivität dargestellt.

In Fig. 5 sind die Werte zur Enzymstabilität bei unterschiedlichen Temperaturen dargestellt. Die Stabilität bei unterschiedlichen Temperaturen wurde im Aktivitätsassay gemessen, nachdem das Enzym in einer Konz. von 2,6 U/mL bei unterschiedlichen Temperaturen für 30 min. in Glycin-Puffer, pH 10, in Abwesenheit von Substrat vorinkubiert wurde.

In Fig. 6 ist die Bleichleistung bezüglich unterschiedlicher Anschmutzungen in Form der Differenz der relativen Helligkeitswerte zwischen Waschmittel und Waschmittel mit enzymatischem Bleichsystem sind dargestellt.

### Sequenzen:

**1. Sorbitoloxidase einer Streptomyces-Spezies "H-7775" (Oda *et al*.)**
**2. Xylitoloxidase einer Streptomyces-Spezies "IKD472" (zu Yamashita *et al*.)** 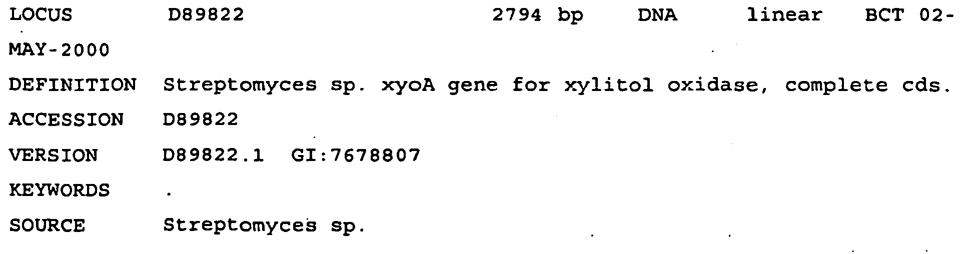 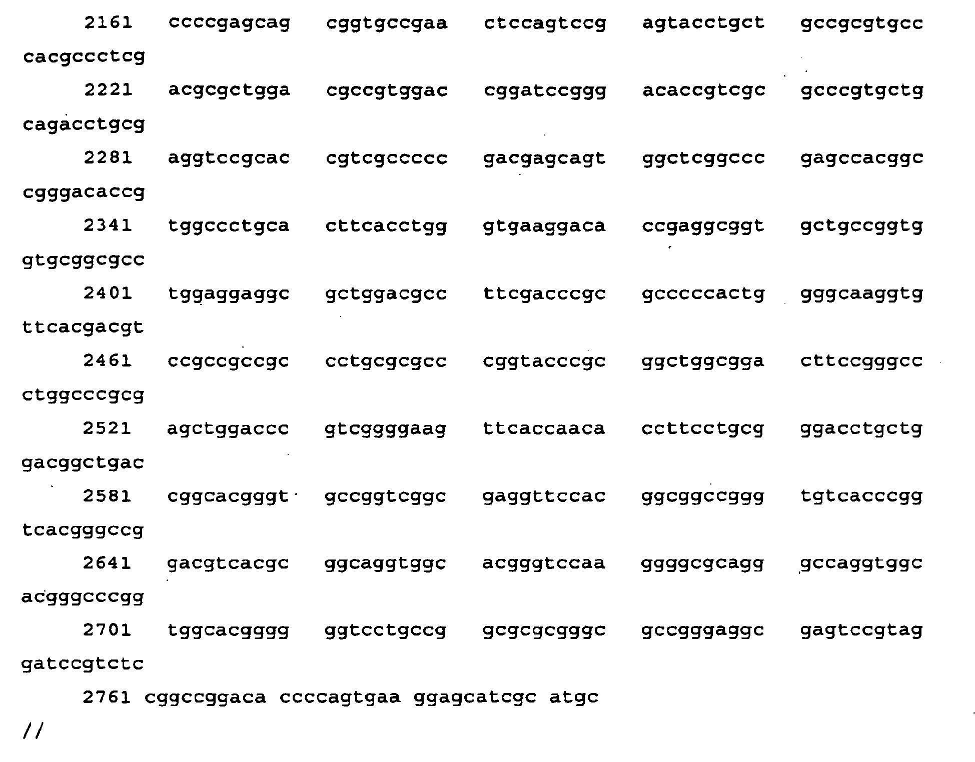
**3. Das im Rahmen dieser Erfindung klonierte Gen aus *S*. *coelicolor* A3 (2) (Wildtypsequenz)** 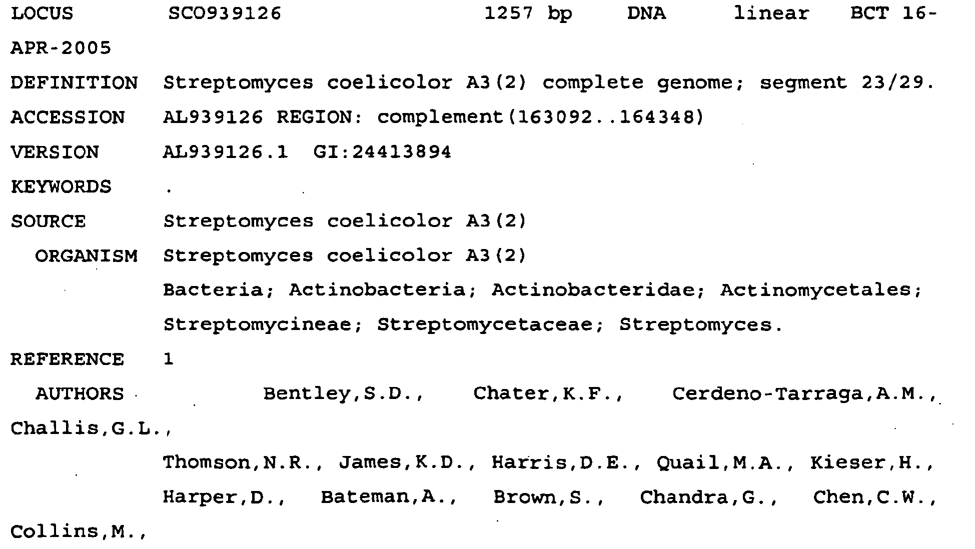
**4. Das im Rahmen dieser Erfindung klonierte Gen aus *S*. *coelicolor* A3(2) (Expressionskonstrukt)**
   **Aminosäuresequenz:**
**5. DNA-Sequenz der codierenden Region:**
**6. PCR-Primer**
   5'-TTAGGATCCATGAGCGACATCACGGTCACCAACTGG-3'
**7. PCR-Primer**
   5'-ATTAAGCTTCCGCGAGCACCCCGCGCACGAACG-3'

## Patentansprüche

1. Körperpflegemittel, Haarpflegemittel, Haarwaschmittel, Süßigkeiten, Mund-, Zahn- und Zahnprothesenpflegemittel, Zahnspangenpflegemittel, Kosmetika, Therapeutika, Waschmittel, Reinigungsmittel, Aufhellungsmittel, Bleichmittel, Desinfektionsmittel, Nachspülmittel, Handwaschmittel, Handgeschirrspülmittel, Maschinengeschirrspülmittel und Mittel zur bleichenden oder desinfizierenden Behandlung von Filtermedien, Textilien, Pelzen, Papier, Fellen oder Leder, enthaltend eine Polyol-Oxidase, insbesondere eine Zuckeralkoholoxidase, deren Aminosäuresequenz mit der in Seq. 4 angegebenen Aminosäuresequenz zu mindestens 85 %, insbesondere mindestens 90 %, besonders bevorzugt mindestens 95 % und ganz besonders bevorzugt zu 100 % übereinstimmt.

2. Mittel nach Anspruch 1, **dadurch gekennzeichnet, dass** das Mittel ein Substrat für die Zuckeralkoholoxidase enthält.

3. Mittel nach Anspruch 1 oder 2, **dadurch gekennzeichnet, dass** es als rieselfähiges Pulver mit einem Schüttgewicht von 300 g/l bis 1200 g/l, insbesondere 500 g/l bis 900 g/l, vorliegt.

4. Mittel nach Anspruch 1 oder 2, **dadurch gekennzeichnet, dass** es in Form eines ein- oder mehrphasigen, insbesondere zweiphasigen flüssigen und versprühbaren Mittels oder eines pastenförmigen oder flüssigen Waschmittels vorliegt.

5. Mittel nach Anspruch 1 oder 2, **dadurch gekennzeichnet, dass** es in Form eines nicht-wäßrigen Flussigwaschmittels oder einer nicht-wässrigen Paste vorliegt.

6. Mittel nach Anspruch 1, 2 oder 5, **dadurch gekennzeichnet, dass** es in Form eines wässrigen Flüssigwaschmittels oder einer wasserhaltigen Paste vorliegt und in einem luftundurchlässigen Behältnis verpackt ist, aus dem es kurz vor Gebrauch oder während des Waschvorgangs freigesetzt wird.

7. Mittel nach einem der Ansprüche 1 bis 6, **dadurch gekennzeichnet, dass** die Polyol-Oxidase und/oder das Substrat für dieses Enzym mit einer bei Raumtemperatur oder bei Abwesenheit von Wasser für das Enzym und/oder dessen Substrat undurchlässigen Substanz umhüllt ist, welche unter Anwendungsbedingungen des Mittels durchlässig für das Enzym und/oder dessen Substrat wird.

8. Mittel nach einem der Ansprüche 1 bis 7, **dadurch gekennzeichnet, dass** es zusätzlich
• 5 Gew.-% bis 70 Gew.-%, insbesondere 10 Gew.-% bis 50 Gew.-% Tensid,
• 10 Gew.-% bis 65 Gew.-%, insbesondere 12 Gew.-% bis 60 Gew. -% wasserlösliches, wasserdispergierbares anorganisches Buildermaterial,
• 1 Gew.-% bis 10 Gew.-%, insbesondere 2 Gew.-% bis 8 Gew.-%, wasserlösliche organische Buildersubstanzen,
• nicht über 15 Gew.-% feste anorganische und/oder organische Säuren beziehungsweise saure Salze,
• nicht über 5 Gew.-% Komplexbildner für Schwermetalle,
• nicht über 5 Gew.-% Vergrauungsinhibitor,
• nicht über 5 Gew. -% Farbübertragungsinhibitor und
• nicht über 5 Gew.-% Schauminhibitor
enthält.

9. Mittel nach einem der vorhergehenden Ansprüche, **dadurch gekennzeichnet, dass** es in einem Mehrkammersystem enthalten' ist, wobei sich Polyol-Oxidase und Polyol in unterschiedlichen Kammern befinden.

10. Verfahren zur Entfernung einer Anschmutzung, **dadurch gekennzeichnet, dass** eine Polyol-Oxidase, insbesondere eine Zuckeralkoholoxidase, deren Aminosäuresequenz mit der in Seq. 4 angegebenen Aminosäuresequenz zu mindestens 85 %, insbesondere mindestens 90 %. besonders bevorzugt mindestens 95 % und ganz besonders bevorzugt zu 100 % übereinstimmt, auf die zu entfernende Anschmutzung aufgebracht wird.

11. Verfahren nach Anspruch 10, **dadurch gekennzeichnet, dass** gleichzeitig oder zeitlich versetzt ein Substrat der Zuckeralkoholoxidase auf die Anschmutzung aufgebracht wird.

12. Verfahren nach Anspruch 11, **dadurch gekennzeichnet, dass** sich Zuckeralkoholoxidase und Substrat der Zuckeralkoholoxidase in unterschiedlichen Kammern eines Mehrkammersystems befinden.

13. Verfahren nach Anspruch 10, **dadurch gekennzeichnet, dass** sich das Substrat der Zuckeralkoholoxidase in der Anschmutzung befindet.

## Claims

1. Body care products, hair care products, hair shampoos, candy, compositions for oral care, dental care and dental prosthesis care, dental brace care products, cosmetics, therapeutics, washing agents, cleaning agents, brightening agents, bleaching agents, disinfectants, rinse agents, detergents for hand washing, dish washing detergents, automatic dishwasher detergents and compositions for bleaching or disinfecting filter media, textiles, furs, paper, hides or leather, comprising a polyol-oxidase, in particular a sugar alcohol-oxidase, whose amino acid sequence corresponds to the amino acid sequence listed in seq. 4 to at least 85 %, in particular to at least 90 %, particularly preferably to at least 95 % and quite particularly preferably to 100 %.

2. The composition according to claim 1, **characterised in that** said composition comprises a substrate for the sugar alcohol-oxidase.

3. The composition according to claim 1 or 2, **characterised in that** said composition is present as a free-flowing powder with a bulk density of 300 g/L to 1200 g/L, particularly 500 g/L to 900 g/L.

4. The composition according to claim 1 or 2, **characterised in that** said composition is present in the form of a single or multi-phase, especially two-phase liquid and sprayable composition or a pasty or liquid washing composition.

5. The composition according to claim 1 or 2, **characterised in that** said composition is present in the form of a non-aqueous liquid washing composition or a non-aqueous paste.

6. The composition according to claim 1, 2 or 5, **characterised in that** said composition is present in the form of an aqueous liquid washing composition or a water-containing paste and is packaged in an airtight container, from which it is released shortly before use or during the wash cycle.

7. The composition according to one of claims 1 to 6, **characterised in that** the polyol-oxidase and/or the substrate for this enzyme is encapsulated with a substance that is impermeable for the enzyme and/or its substrate at room temperature or in the absence of water, and which becomes permeable for the enzyme and/or its substrate under the conditions of use of the composition.

8. The composition according to one of claims 1 to 7, **characterised in that** it additionally comprises
• 5 wt % to 70 wt %, particularly 10 wt % to 50 wt % surfactant,
• 10 wt % to 65 wt %, particularly 12 wt % to 60 wt % of water-soluble, water-dispersible inorganic builder,
• 1 wt % to 10 wt %, particularly 2 wt % to 8 wt %, of water-soluble organic builder substances,
• not more than 15 wt % solid inorganic and/or organic acids or acid salts,
• not more than 5 wt % sequestrants for heavy metals,
• not more than 5 wt % greying inhibitor,
• not more than 5 wt % colour transfer inhibitor and
• not more than 5 wt % foam inhibitor.

9. The composition according to one of the previous claims, **characterised in that** said composition is comprised in a multi-chamber system, wherein polyol-oxidase and polyol are present in different chambers.

10. Method for removing a soil, **characterised in that** a polyol-oxidase, in particular a sugar alcohol-oxidase, whose amino acid sequence corresponds to at least 85 % to the amino acid sequence listed in seq. 4, in particular to at least 90 %, particularly preferably to at least 95 % and quite particularly preferably to 100 %, is applied onto the soil to be removed.

11. The method according to claim 10, **characterised in that** a substrate of the sugar alcohol-oxidase is applied simultaneously or at a different time onto the soil.

12. The method according to claim 11, **characterised in that** sugar alcohol-oxidase and substrate of the sugar alcohol-oxidase are present in different chambers of a multi-chamber system.

13. The method according to claim 10, **characterised in that** the substrate of the sugar alcohol-oxidase is present in the soil.

## Revendications

1. Agent de soin corporel, agent de soin capillaire, agent de lavage de cheveux, sucreries, agent de soin buccal, dentaire et de prothèse dentaire, agent de soin d'appareil dentaire, cosmétique, thérapeutique, agent de lavage, agent de nettoyage, agent d'éclaircissement, agent de blanchiment, agent désinfectant, agent de post-rinçage, agent de lavage des mains, agent de lavage à la main de vaisselle, détergent pour lave-vaisselle et agent pour le traitement blanchissant ou désinfectant d'agents de filtration, de textiles, de fourrures, de papier, de peaux ou de cuir, contenant une polyol-oxydase, en particulier une alcool de sucre-oxydase, dont la séquence d'acides aminés correspond à la séquence d'acides aminés indiquée dans la SEQ. 4 à raison d'au moins 85%, en particulier d'au moins 90%, de manière particulièrement préférée d'au moins 95% et de manière tout particulièrement préférée à 100%.

2. Agent selon la revendication 1, **caractérisé en ce que** l'agent contient un substrat pour l'alcool de sucre-oxydase.

3. Agent selon la revendication 1 ou 2, **caractérisé en ce qu'**il se trouve sous forme d'une poudre pouvant s'écouler présentant une densité apparente de 300 g/l à 1200 g/l, en particulier de 500 g/l à 900 g/l.

4. Agent selon la revendication 1 ou 2, **caractérisé en ce qu'**il se trouve sous forme d'un agent à une ou plusieurs phases, en particulier à deux phases, liquide et pulvérisable ou sous forme d'un agent de lavage sous forme de pâte ou liquide.

5. Agent selon la revendication 1 ou 2, **caractérisé en ce qu'**il se trouve sous forme d'un agent de lavage liquide non aqueux ou d'une pâte non aqueuse.

6. Agent selon la revendication 1, 2 ou 5, **caractérisé en ce qu'**il se trouve sous forme d'un agent de lavage liquide aqueux ou d'une pâte contenant de l'eau et est emballé dans un récipient imperméable à l'air, dont il est libéré peu avant l'emploi ou pendant le cycle de lavage.

7. Agent selon l'une quelconque des revendications 1 à 6, **caractérisé en ce que** la polyol-oxydase et/ou le substrat pour cette enzyme est enrobée d'une substance imperméable à l'enzyme et/ou à son substrat à température ambiante ou en l'absence de l'eau, qui devient perméable à l'enzyme et/ou à son substrat dans les conditions d'utilisation de l'agent.

8. Agent selon l'une quelconque des revendications 1 à 7, **caractérisé en ce qu'**il contient en outre
- 5% en poids à 70% en poids, en particulier 10% en poids à 50% en poids d'agent tensioactif,
- 10% en poids à 65% en poids, en particulier 12% en poids à 60% en poids de builder inorganique soluble dans l'eau, dispersible dans l'eau,
- 1 % en poids à 10% en poids, en particulier 2% en poids à 8% en poids de substances builders organiques, solubles dans l'eau,
- pas plus de 15% en poids d'acides ou, selon le cas, de sels acides solides, inorganiques et/ou organiques,
- pas plus de 5% en poids de complexants pour métaux lourds,
- pas plus de 5% en poids d'inhibiteur de grisaillement,
- pas plus de 5% en poids d'inhibiteur de transfert de couleur et
- pas plus de 5% en poids d'inhibiteur de mousse.

9. Agent selon l'une quelconque des revendications précédentes, **caractérisé en ce qu'**il est contenu dans un système à plusieurs compartiments, la polyol-oxydase et le polyol se trouvant dans des compartiments séparés.

10. Procédé pour éliminer une souillure, **caractérisé en ce qu'**une polyol-oxydase, en particulier une alcool de sucre-oxydase, dont la séquence d'acides aminés correspond à la séquence d'acides aminés indiquée dans la SEQ. 4 à raison d'au moins 85%, en particulier d'au moins 90%, de manière particulièrement préférée d'au moins 95% et de manière tout particulièrement préférée à 100%, est appliquée sur la souillure à éliminer.

11. Procédé selon la revendication 10, **caractérisé en ce qu'**on applique simultanément ou de manière décalée dans le temps un substrat de l'alcool de sucre-oxydase sur la souillure.

12. Procédé selon la revendication 11, **caractérisé en ce que** l'alcool de sucre-oxydase et le substrat de l'alcool de sucre-oxydase se trouvent dans des compartiments différents d'un système à plusieurs chambres.

13. Procédé selon la revendication 10, **caractérisé en ce que** le substrat de l'alcool de sucre-oxydase se trouve dans la souillure.


### SEQUENCE LISTING

<110> Henkel Kommanditgesellschaft auf Aktien
<120> System zur enzymatischen Generierung von Wasserstoffperoxid
<130> H 06747 PCT
<141> 2005-11-08
<160> 7
<170> SeqWin99, version 1.02
<210> 1
   <211> 1557
   <212> DNA
   <213> Streptomyces sp.
<220>
   <223> Sorbitoloxidase einer Streptomyces-Spezies
<400> 1
   ggatcccatg ttctgtgtgc acctgttggt atgtgtgaca cgcgccaaac ggttggtgtg   60
   cggatatgta cctcggtccg caactaccgt acgtgctcgt gcggttgcat gggtggtttc   120
   gggccgctcg gctccggcgg ccgcggggcg gtctgggaga atgcagtgcc gggagcacgt   180
   gcccggccat gagtggaaga aggacgcacg atgacccccg cggagaagaa ctgggccggc   240
   aacatcacct tcggcgcgaa gcggctgtgt gtgccgagat ccgtccggga actgcgcgag   300
   acggtcgccg cctccggcgc ggtccgcccc ctggggaccc ggcactcctt caacaccgtc   360
   gcggacacct ccggcgacca tgtgtcgctc gccggactcc cgcgcgtcgt ggacatcgac   420
   gtcccgggcc gcgccgtctc gctgtccgcc ggactccgct tcggggagtt cgccgccgaa   480
   ctgcacgcgc gcggtctcgc cctcgccaac ctgggctcgc tcccgcacat ctcggtcgcc   540
   ggcgccgtcg cgaccggcac ccacggctcg ggcgtgggca accgctcatt ggcgggcgcg   600
   gtgcgtgccc tctccctggt gacggccgac ggggagacgc gcaccctgcg gcgcaccgac   660
   gaggacttcg cgggcgcggt cgtctccctc ggcgccctcg gcgtggtgac gtcgctggaa   720
   ctcgacctcg tgcccgcctt cgaggtgcgc cagtgggtgt acgaggacct gcccgaggcg   780
   acactcgccg cgcgcttcga cgaggtgatg tccgccgcct acagcgtcag cgtcttcacc   840
   gactggcgcc ccggcccggt cggccaggtc tggctcaagc agcgggtggg cgacgagggg   900
   gcacggtccg tgatgcccgc cgagtggctg ggcgcccggc tcgccgacgg cccccggcac   960
   ccggtccccg ggatgcccgc cgggaactgc acggcgcagc agggggtgcc cgggccctgg   1020
   cacgagcggc ttccgcactt ccggatggag ttcaccccga gcaacggcga cgagctccag   1080
   tcggagtact tcgtggcccg cgcggacgcc gtcgccgcct acgaggccct ggcccggctg   1140
   cgggaccgga tcgccccggt gctccaggtc tccgagctcc gcaccgtcgc cgccgacgac   1200
   ctgtggctga gccccgccca cggccgggac tcggtggcct tccacttcac ctgggtcccg   1260
   gacgcggcgg ccgtcgcgcc ggtggccggg gcgatcgagg aggccctggc cccgttcggc   1320
   gcccgcccgc actggggcaa ggtcttctcg acggcccccg aggtgctgcg cacgctctac   1380
   ccgcgctacg cggacttcga ggagctggtg ggccgccacg accccgaggg caccttccgc   1440
   aacgcgttcc tggaccggta cttccgccgc tgatccgcgg gctcagcgac cgtcgcgtcc   1500
   gtcctcggcc cgggacggcc aggcggcggc cgagcggccc ggggcgagct ggtcgac      1557
<210> 2
   <211> 2794
   <212> DNA
   <213> Streptomyces sp.
<220>
   <223> Xylitoloxidase einer Streptomyces-Spezies
<400> 2
<210> 3
   <211> 1257
   <212> DNA
   <213> Streptomyces coelicolor
<220>
   <223> Das im Rahmen der vorliegenden Erfindung klonierte Gen aus S. coelicolor
<400> 3
   atgagcgaca tcacggtcac caactgggcc ggcaacatca cgtacacggc gaaggaactg   60
   ctgcggccgc actccctgga cgcgctgcgg gccctggtgg cggacagcgc cagggtgcgg  120
   gtgctgggca gcgggcactc cttcaacgag atcgccgagc cgggcgacgg gggtgtcctg  180
   ctgtcgctgg cgggcctgcc gtccgtggtg gacgtggaca cggcggcccg tacggtgcgg   240
   gtcggcggcg gtgtgcggta cgcggagctg gcccgggtgg tgcacgcgcg gggcctggcg   300
   ctgccgaaca tggcctcgct gccgcacatc tcggtcgccg ggtcggtggc caccggcacc   360
   cacggttcgg gggtgggcaa cggttcgctg gcctcggtgg tgcgcgaggt ggagctggtc   420
   accgcggacg gttcgaccgt ggtgatcgcg cggggcgacg agcggttcgg cggggcggtg   480
   acctcgctcg gcgcgctggg cgtggtgacg tcgctcacac tcgacctgga gccggcgtac   540
   gagatggaac agcacgtctt caccgagctg ccgctggccg ggttggaccc ggcgacgttc   600
   gagacggtga tggcggcggc gtacagcgtg agtctgttca ccgactggcg ggcgcccggt   660
   ttccggcagg tgtggctgaa gcggcgcacc gaccggccgc tggacggttt cccgtacgcg   720
   gccccggccg ccgagaagat gcatccggtg ccgggcatgc ccgcggtgaa ctgcacggag   780
   cagttcgggg tgccggggcc ctggcacgag cggctgccgc acttccgcgc ggagttcacg   840
   cccagcagcg gtgccgagtt gcagtcggag tacctgatgc cccgggagca cgccctggcc   900
   gccctgcacg cgatggacgc gatacgggag acgctcgcgc cggtgctcca gacctgcgag   960
   atccgcacgg tcgccgccga cgcgcagtgg ctgagcccgg cgtacgggcg ggacaccgtg  1020
   gccgcgcact tcacctgggt cgaggacacg gcggcggtgc tgccggtggt gcggcggctg  1080
   gaggaggcgc tcgtcccctt cgcggcccgt ccgcactggg ggaaggtgtt caccgtcccg  1140
   gcgggcgagc tgcgtgcgct gtacccgcgg ctggccgact tcggggcgct ggccggggcg  1200
   ctggacccgg cggggaagtt caccaacgcg ttcgtgcgcg gggtgctcgc gggctga     1257
<210> 4
   <211> 431
   <212> PRT
   <213> Streptomyces coelicolor
<220>
   <223> Das im Rahmen dieser Erfindung klonierte Gen aus S. coelicolor A3(2) (Expressionskonstrukt)
<400> 4
   Met Arg Gly Ser His His His His His His Gly Ser Met Ser Asp Ile
   1               5                   10                  15
   Thr Val Thr Asn Trp Ala Gly Asn Ile Thr Tyr Thr Ala Lys Glu Leu
               20                  25                  30
   Leu Arg Pro His Ser Leu Asp Ala Leu Arg Ala Leu Val Ala Asp Ser
           35                  40                  45
   Ala Arg Val Arg Val Leu Gly Ser Gly His Ser Phe Asn Glu Ile Ala
       50                  55                  60
   Glu Pro Gly Asp Gly Gly Val Leu Leu Ser Leu Ala Gly Leu Pro Ser
   65                  70                  75                  80
   Val Val Asp Val Asp Thr Ala Ala Arg Thr Val Arg Val Gly Gly Gly
                   85                  90                  95
   Val Arg Tyr Ala Glu Leu Ala Arg Val Val His Ala Arg Gly Leu Ala
               100                 105                 110
   Leu Pro Asn Met Ala Ser Leu Pro His Ile Ser Val Ala Gly Ser Val
           115                 120                 125
   Ala Thr Gly Thr His Gly Ser Gly Val Gly Asn Gly Ser Leu Ala Ser
       130                 135                 140
   Val Val Arg Glu Val Glu Leu Val Thr Ala Asp Gly Ser Thr Val Val
   145                 150                 155                 160
   Ile Ala Arg Gly Asp Glu Arg Phe Gly Gly Ala Val Thr Ser Leu Gly
                   165                 170                 175
   Ala Leu Gly Val Val Thr Ser Leu Thr Leu Asp Leu Glu Pro Ala Tyr
               180                 185                 190
   Glu Met Glu Gln His Val Phe Thr Glu Leu Pro Leu Ala Gly Leu Asp
           195                 200                 205
   Pro Ala Thr Phe Glu Thr Val Met Ala Ala Ala Tyr Ser Val Ser Leu
       210                 215                 220
   Phe Thr Asp Trp Arg Ala Pro Gly Phe Arg Gln Val Trp Leu Lys Arg
   225                 230                 235                 240
   Arg Thr Asp Arg Pro Leu Asp Gly Phe Pro Tyr Ala Ala Pro Ala Ala
                   245                 250                 255
   Glu Lys Met His Pro Val Pro Gly Met Pro Ala Val Asn Cys Thr Glu
               260                 265                 270
   Gln Phe Gly Val Pro Gly Pro Trp His Glu Arg Leu Pro His Phe Arg
           275                 280                 285
   Ala Glu Phe Thr Pro Ser Ser Gly Ala Glu Leu Gln Ser Glu Tyr Leu
       290                 295                 300
   Met Pro Arg Glu His Ala Leu Ala Ala Leu His Ala Met Asp Ala Ile
   305                 310                 315                 320
   Arg Glu Thr Leu Ala Pro Val Leu Gln Thr Cys Glu Ile Arg Thr Val
                   325                 330                 335
   Ala Ala Asp Ala Gln Trp Leu Ser Pro Ala Tyr Gly Arg Asp Thr Val
               340                 345                 350
   Ala Ala His Phe Thr Trp Val Glu Asp Thr Ala Ala Val Leu Pro Val
           355                 360                 365
   Val Arg Arg Leu Glu Glu Ala Leu Val Pro Phe Ala Ala Arg Pro His
       370                 375                 380
   Trp Gly Lys Val Phe Thr Val Pro Ala Gly Glu Leu Arg Ala Leu Tyr
   385                 390                 395                 400
   Pro Arg Leu Ala Asp Phe Gly Ala Leu Ala Gly Ala Leu Asp Pro Ala
                   405                 410                 415
   Gly Lys Phe Thr Asn Ala Phe Val Arg Gly Val Leu Ala Glu Ala
               420                 425                 430
<210> 5
   <211> 1296
   <212> DNA
   <213> Streptomyces coelicolor
<220>
   <223> DNA-Sequenz der codierenden Region
<400> 5
   atgagaggat cgcatcacca tcaccatcac ggatccatga gcgacatcac ggtcaccaac   60
   tgggccggca acatcacgta cacggcgaag gaactgctgc ggccgcactc cctggacgcg  120
   ctgcgggccc tggtggcgga cagcgccagg gtgcgggtgc tgggcagcgg gcactccttc  180
   aacgagatcg ccgagccggg cgacgggggt gtcctgctgt cgctggcggg cctgccgtcc  240
   gtggtggacg tggacacggc ggcccgtacg gtgcgggtcg gcggcggtgt gcggtacgcg  300
   gagctggccc gggtggtgca cgcgcggggc ctggcgctgc cgaacatggc ctcgctgccg  360
   cacatctcgg tcgccgggtc ggtggccacc ggcacccacg gttcgggggt gggcaacggt  420
   tcgctggcct cggtggtgcg cgaggtggag ctggtcaccg cggacggttc gaccgtggtg  480
   atcgcgcggg gcgacgagcg gttcggcggg gcggtgacct cgctcggcgc gctgggcgtg  540
   gtgacgtcgc tcacactcga cctggagccg gcgtacgaga tggaacagca cgtcttcacc  600
   gagctgccgc tggccgggtt ggacccggcg acgttcgaga cggtgatggc ggcggcgtac  660
   agcgtgagtc tgttcaccga ctggcgggcg cccggtttcc ggcaggtgtg gctgaagcgg  720
   cgcaccgacc ggccgctgga cggtttcccg tacgcggccc cggccgccga gaagatgcat  780
   ccggtgccgg gcatgcccgc ggtgaactgc acggagcagt tcggggtgcc ggggccctgg  840
   cacgagcggc tgccgcactt ccgcgcggag ttcacgccca gcagcggtgc cgagttgcag  900
   tcggagtacc tgatgccccg ggagcacgcc ctggccgccc tgcacgcgat ggacgcgata  960
   cgggagacgc tcgcgccggt gctccagacc tgcgagatcc gcacggtcgc cgccgacgcg 1020
   cagtggctga gcccggcgta cgggcgggac accgtggccg cgcacttcac ctgggtcgag 1080
   gacacggcgg cggtgctgcc ggtggtgcgg cggctggagg aggcgctcgt ccccttcgcg 1140
   gcccgtccgc actgggggaa ggtgttcacc gtcccggcgg gcgagctgcg tgcgctgtac 1200
   ccgcggctgg ccgacttcgg ggcgctggcc ggggcgctgg acccggcggg gaagttcacc 1260
   aacgcgttcg tgcgcggggt gctcgcggaa gcttaa                           1296
<210> 6
   <211> 36
   <212> DNA
   <213> Streptomyces coelicolor
<220>
   <223> PCR-Primer
<400> 6
   ttaggatcca tgagcgacat cacggtcacc aactgg 36
<210> 7
   <211> 33
   <212> DNA
   <213> Streptomyces coelicolor
<220>
   <223> PCR-Primer
<400> 7
   attaagcttc cgcgagcacc ccgcgcacga acg 33